(19) Europäisches Patentamt
European Patent Office
Office européen des brevets

(11) EP 1 258 247 A1

(12) EUROPEAN PATENT APPLICATION

(43) Date of publication:
20.11.2002 Bulletin 2002/47

(51) Int Cl.[7]: **A61K 31/52**, A61P 3/10

(21) Application number: **01111651.4**

(22) Date of filing: **14.05.2001**

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **Aventis Pharma Deutschland GmbH**
**65929 Frankfurt am Main (DE)**

(72) Inventors:
- **Herling, Andreas, Dr.**
  **65520 Bad Camberg (DE)**
- **Jähne, Gerhard, Dr.**
  **65929 Frankfurt (DE)**
- **Maguire, Martin P.**
  **Cambridge, Massachussetts 02141 (US)**
- **Spada, Alfred P.**
  **Carlsbad, California 92009 (US)**
- **Myers, Michael R.**
  **Fishers, Indiana 46038 (US)**
- **Choi-Sledeski, Yong Mi**
  **Belle Mead, New Jersey 08502 (US)**
- **Pauls, Heinz W.**
  **Flemington, New Jersey 08822 (US)**
- **Ewing, William R.**
  **Yardley, Pennsylvania 19067 (US)**

(74) Representative: **Isenbruck, Günter, Dr. et al**
**Patent- und Rechtsanwälte,**
**Bardehle-Pagenberg-Dost-Altenburg-Geissler-Isenbruck**
**Theodor-Heuss-Anlage 12**
**68165 Mannheim (DE)**

# (54) Adenosine analogues for the treatment of insulin resistance syndrome and diabetes

(57) The invention relates to the use of adenosine compounds described by formula I

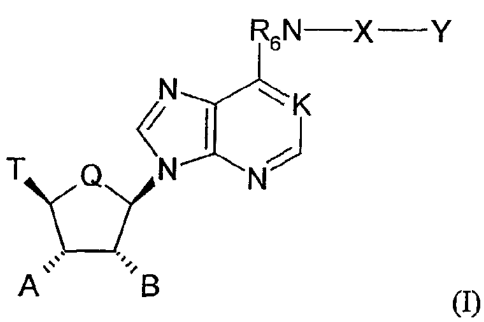

(I)

and certain derivatives thereof for producing a medicine for the treatment of the insulin resistance syndrome and diabetes.

In the formula I
K is N, N→O, or CH;
Q is $CH_2$ or O;
$R_6$ is hydrogen, alkyl, allyl, 2-methallyl, 2-butenyl, or cycloalkyl;

X is 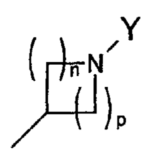 or 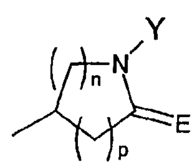

E is O or S;
Y is e.g. hydrogen, alkyl, aralkyl, aryl;
n and p are independently 0, 1, 2, or 3, provided that n + p is at least 1;
T is e.g hydrogen, alkyl, acyl, thioacyl, halo, carboxyl;
$R_1$, $R_2$ and $R_3$ are independently H, alkyl, or cycloalkyl;
A is hydrogen, alkyl, hydroxyalkyl, alkoxyalkyl, or OR';
B is hydrogen, alkyl, hydroxyalkyl, alkoxyalkyl, or OR";
R' and R" are e.g independently hydrogen, alkyl, aralkyl or carbamoyl.

EP 1 258 247 A1

## Description

The use of adenosine and analogues for producing medicines for the treatment of the insulin resistance syndrome and diabetes

This invention relates to the use of compounds derived from adenosine and analogues thereof as insulin resistance agents.

Adenosine has a wide variety of physiological and pharmacological actions including a marked alteration of cardiovascular and renal function. In animals and man, intravenous injection of the adenosine nucleotide causes hypotension.

The physiological and pharmacological actions of adenosine are mediated through specific receptors located on cell surfaces. Four adenosine receptor subtypes, designated as $A_1$, $A_{2A}$, $A_{2B}$, and $A_3$ receptors, have been identified. The $A_1$ receptor inhibits the formation of cAMP by suppressing the activity of adenylate cyclase, while stimulation of $A_2$ receptors increases adenylate cyclase activity and intracellular CAMP. Each receptor appears to mediate specific actions of adenosine in different tissues: for example, the vascular actions of adenosine appears to be mediated through stimulation of $A_2$ receptors, which is supported by the positive correlation between cAMP generation and vasorelaxation in adenosine-treated isolated vascular smooth muscle; while stimulation of the cardiac $A_1$ receptors reduces cAMP generation in the heart which contributes to negative dromotropic, inotropic and chronotropic cardiac effects. Consequently, unlike most vasodilators, adenosine administration does not produce a reflex tachycardia.

Adenosine also exerts a marked influence on renal function. Intrarenal infusion of adenosine causes a transient fall in renal blood flow and an increase in renal vascular resistance. With continued infusion of adenosine, renal blood flow returns to control levels and renal vascular resistance is reduced. The initial renal vasoconstrictor responses to adenosine are not due to direct vasoconstrictor actions of the nucleotide, but involve an interaction between adenosine and the renin-angiotensin system.

Adenosine is widely regarded as the primary physiological mediator of reactive hyperemia and autoregulation of the coronary bed in response to myocardial ischemia. It has been reported that the coronary endothelium possesses adenosine $A_2$ receptors linked to adenylate cyclase, which are activated in parallel with increases in coronary flow and that cardiomyocyte receptors are predominantly of the adenosine $A_1$ subtype and associated with bradycardia. Accordingly, adenosine offers a unique mechanism of ischemic therapy.

Cardiovascular responses to adenosine are short-lived due to the rapid uptake and metabolism of the endogenous nucleotide. In contrast, the adenosine analogues are more resistant to metabolic degradation and are reported to elicit sustained alterations in arterial pressure and heart rate.

Adenosine compounds useful as anti-hypertensive, cardioprotective, anti-ischemic and antilipolytic agents are known from EP A 0 912 520 (HOE 1996/S 013).

Several potent metabolically-stable analogues of adenosine have been synthesized which demonstrate varying degrees of selectivity for the two receptor subtypes. Adenosine agonists have generally shown greater selectivity for $A_1$ receptors as compared to $A_2$ receptors. Cyclopentyladenosine (CPA) and R-phenylisopropyl-adenosine (R-PIA) are standard adenosine agonists which show marked selectivity for the $A_1$ receptor ($A_2/A_1$ ratio = 780 and 106, respectively). In contrast, N-5'-ethyl-carboxamido adenosine (NECA) is a potent $A_2$ receptor agonist (Ki-12 nM) but has equal affinity for the $A_1$ receptor (Ki-6.3 nM; $A_2/A_1$ ratio = 1.87). Until recently, CV-1808 was the most selective $A_2$ agonist available ($A_2/A_1$ =0.19), even though the compound was 10-fold less potent than NECA in its affinity for the $A_2$ receptor. In recent developments, newer compounds have been disclosed which are very potent and selective $A_2$ agonists (Ki=3-8 nM for $A_1$; $A_2/A_1$ ratio=0.027-0.042) (C.E. Müller and T. Scior, *Pharmaceutica Acta Hevetiae* 68 (1993) 77-111).

Various N6-aryl and N6-heteroarylalkyl substituted adenosines, and substituted-(2-amino and 2-hydroxy)adenosines, have been reported in the literature as possessing varied pharmacological activity, including cardiac and circulatory activity. See, for example, British Patent Specification 1,123,245, German Offen. 2,136,624, German Off 2,059,922, German Offen. 2,514,284, South African Patent No. 67/7630, U.S. Patent No. 4,501,735, EP Publication No. 0139358 (disclosing N6-[geminal diaryl substiuted alkyl]adenosines), EP Patent Application Ser. No. 88106818.3 (disclosing that N6-heterocyclic-substituted adenosine derivatives exhibit cardiac vasodilatory activity), German Offen. 2,131,938 (disclosing aryl and heteroaryl alkyl hydrazinyl adenosine derivatives), German Offen. 2,151,013 (disclosing N6-aryl and heteroaryl substituted adenosines), German Offen. 2,205,002 (disclosing adenosines with N6-substituents comprising bridged ring structures linking the N6-nitrogen to substituents including thienyl) and South African Patent No. 68/5477 (disclosing N6-indolyl substituted-2-hydroxy adenosines).

U.S. Patent No. 4,954,504 and EP Publication No. 0267878 disclose generically that carbocyclic ribose analogues of adenosine, and pharmaceutically acceptable esters thereof, substituted in the 2- and/or N6-positions by aryl lower alkyl groups including thienyl, tetrahydropyranyl, tetrahydrothiopyranyl, and bicyclic benzo fused 5- or 6-membered saturated heterocyclic lower alkyl derivatives exhibit adenosine receptor agonist properties. Adenosine analogues having thienyl-type substituents are described in EP Publication No. 0277917 (disclosing N6-substituted-2-heteroarylalkylamino substituted adenosines including 2-[(2-[thien-2-yl]ethyl)amino] substituted adenosine), German Offenlegung 2,139,107 (disclosing N6-[benzothienylmethyl]-adenosine), PCT WO 85/04882 (disclosing that N6-heterocyclicalkyl-substituted adenosine derivatives, including N6-[2-(2-thienyl)ethyl]amino-9-(D-ribofuranosyl)-9H-purine, exhibit cardiovascular vasodilatory activity and that N6-chiral substituents exhibit enhanced activity), EP Published Application No. 0232813 (disclosing that N6-(1-substituted thienyl)cyclopropylmethyl substituted adenosines exhibit cardiovascular activity), U.S. Patent No. 4,683,223 (disclosing that N6-benzothiopyranyl substituted adenosines exhibit antihypertensive properties), PCT WO 88/03147 and WO 88/03148 (disclosing that N6-[2-aryl-2-(thien-2-yl)]ethyl substituted adenosines exhibit antihypertensive properties), U.S. Patent Nos. 4,636,493 and 4,600,707 (disclosing that N6-benzothienylethyl substituted adenosines exhibit antihypertensive properties).

Adenosine-5'-carboxylic acid amides are disclosed as having utility as anti-hypertensive and anti-anginal agents in U.S. Patent No. 3,914,415, while U.S. Patent No. 4,738,954 discloses that N6-substituted aryl and arylalkyl-adenosine 5'-ethyl carboxamides exhibit various cardiac and antihypertensive properties.

$N^6$-alkyl-2'-O-alkyl adenosines are disclosed in EP Publication No. 0,378,518 and UK Patent Application No. 2,226,027 as having antihypertensive activity. $N^6$-alkyl-2',3'-di-O-alkyl adenosines are also reported to have utility as antihypertensive agents, U.S. Patent 4,843,066.

Adenosine-5'-(N-substituted)carboxamides and carboxylate esters and N1-oxides thereof are reported to be coronary vasodilators, Stein, et al., *J. Med. Chem.* 1980, 23, 313-319 and *J. Med. Chem.* 19 (10), 1180 (1976). Adenosine-5'-carboxamides and N1-oxides thereof are also reported as small animal poisons in U.S. Patent No. 4,167,565.

N6-substituted adenosines and analogues, useful in treating gastroinstestinal motility disorders, have been reported in EP Published Applications Nos. 0423776, and 0423777.

N6-heterocyclyl compounds derived from adenosine and analogues thereof, and their use in treating hypertension and myocardial ischemia, their use as cardioprotective agents which ameliorate ischemic injury or myocardial infarct size consequent to myocardial ischemia, their use as antilipolytic agents which reduce plasma lipid levels, serum triglyceride levels, and plasma cholesterol levels, are disclosed in EP-A 0 758 897 (HOE 1994/S 049). N6-heterocyclyl compounds derived from adenosine and analogues thereof, and their use in treating myocardial ischemia and hypertension, are also disclosed in U.S. Patent No. 5,364,862, filed October 2, 1992.

It is believed that the reported toxicity, CNS properties and heart rate elevation associated with adenosine analogueues have contributed to the difficulties preventing the development of a commercial adenosine analogue antihypertensive/antiischemic agent. The present invention relates to a class of metabolically stable adenosine analogues, and derivatives thereof, possessing unexpectedly desirable pharmacological properties, i.e. are anti-hypertensive, cardioprotective, anti-ischemic, and antilipolytic agents having a unique therapeutic profile.

Surprisingly it has been found that adenosine compounds by stimulating adenosine receptor $A_1$ on adipocytes can inhibit the peripheral lipolysis of stored triglycerides and thus can cause an immediate fall in plasma free fatty acids. This antilipolytic effect caused an immediate onset of improvement of insulin sensitivity in insulin resistant mammals, including man. Therefore adenosine agonists are useful as therapeutics to treat the insulin resistant syndrome as well as antidiabetics in insulin resistant diabetics.

The invention relates to the use of adenosine compounds described by Formula I

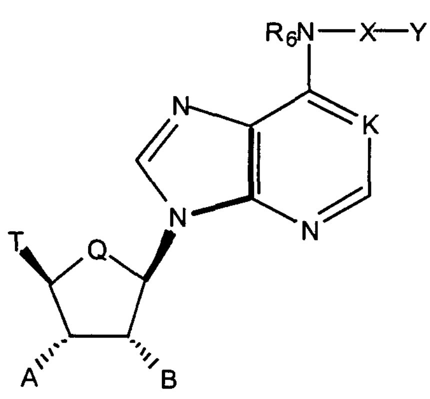

Formula I

wherein:

K is N,N→O, or CH;
Q is $CH_2$ or O;

$R_6$ is hydrogen, alkyl, allyl, 2-methylallyl,2-butenyl, or cycloalkyl;
X is

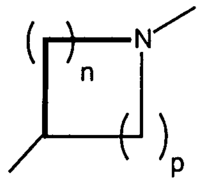
or
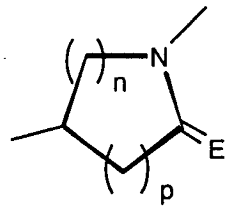

where the nitrogen of the ring of X is substituted by Y;
E is O or S;
Y is hydrogen, alkyl, aralkyl, substituted aralkyl, aryl, substituted aryl, heterocyclyl, substituted heterocyclyl, heterocyclylalkyl, or substituted heterocyclylalkyl;
n and p are independently 0, 1, 2, or 3, provided that n + p is at least 1;
T is hydrogen, alkyl, acyl, thioacyl, halo, carboxyl,

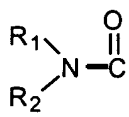
,
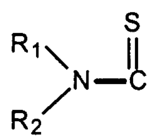

or $R_3O-CH_2$;
$R_1$, $R_2$, and $R_3$ are independently H, alkyl, or cycloalkyl;
A is hydrogen, alkyl, hydroxyalkyl, alkoxyalkyl, or OR';
B is hydrogen, alkyl, hydroxyalkyl, alkoxyalkyl, or OR'';
R' and R'' are independently hydrogen, alkyl, aralkyl, carbamoyl, alkyl carbamoyl, dialkylcarbamoyl, acyl, alkoxycarbonyl, aralkoxycarbonyl, aryloxycarbonyl, or, when A and B are OR' and OR'', respectively, R' and R'' together may form

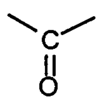
,
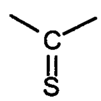
,
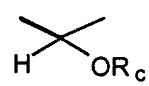

where $R_c$ is hydrogen or alkyl,

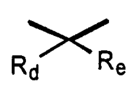

where $R_d$ and $R_e$ are independently hydrogen, alkyl, or together with the carbon atom to which they are attached may form a 1,1-cycloalkyl group;

or a pharmaceutically acceptable salt thereof, pharmaceutically acceptable prodrug thereof, an N-oxide thereof, a hydrate thereof or a solvate thereof for producing a medicine for the treatment of the insulin resistance syndrome and diabetes.

As used above and throughout the description of the invention, the following terms, unless otherwise indicated, shall be understood to have the following meanings:

"Acyl" means a straight or branched alkyl-C=O group. "Thioacyl" means a straight or branched alkyl-C=S group. Preferred acyl and thioacyl groups are lower alkanoyl and lower thioalkanoyl having from 1 to about 6 carbon

atoms in the alkyl group.

"Alkyl" means a saturated aliphatic hydrocarbon group which may be straight or branched and having about 1 to about 20 carbon atoms in the chain. Preferred alkyl groups may be straight or branched and have about 1 to about 10 carbon atoms in the chain. Branched means that a lower alkyl group such as methyl, ethyl or propyl is attached to a linear alkyl chain.

"Lower alkyl" means an alkyl group having 1 to about 6 carbons.

"Cycloalkyl" means an aliphatic ring having 3 to about 10 carbon atoms in the ring. Preferred cycloalkyl groups have 4 to about 7 carbon atoms in the ring.

"Carbamoyl" means an

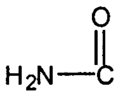

group. Alkylcarbamoyl and dialkylcarbamoyl means that the nitrogen of the carbamoyl is substituted by one or two alkyl groups, respectively.

"Carboxyl" means a COOH group.

"Alkoxy" means an alkyl-O group in which "alkyl" is as previously described. Lower alkoxy groups are preferred. Exemplary groups include methoxy, ethoxy, n-propoxy, i-propoxy and n-butoxy. j

"Alkoxyalkyl" means an alkyl group, as previously described, substituted by an alkoxy group, as previously described.

"Alkoxycarbonyl means an alkoxy-C=O group.

"Aralkyl" means an alkyl group substituted by an aryl radical, wherein "aryl" means a phenyl or naphthyl. "Substituted aralkyl" and "substituted aryl" means that the aryl group, or the aryl group of the aralkyl group is substituted with one or more substituents which include alkyl, alkoxy, amino, nitro, carboxy, carboalkoxy, cyano, alkyl amino, halo, hydroxy, hydroxyalkyl, mercaptyl, alkylmercaptyl, trihaloalkyl, carboxyalkyl or carbamoyl.

"Aralkoxycarbonyl" means an aralkyl-O-C=O group.

"Aryloxycarbonyl" means an aryl-O-C=O group.

"Carbalkoxy" means a carboxyl substituent esterified with an alcohol of the formula $C_nH_{2n+1}OH$, wherein n is from 1 to about 6.

"Halogen" (or "halo") means chlorine (chloro), fluorine (fluoro), bromine (bromo) or iodine (iodo).

"Heterocyclyl" means about a 4 to about a 10 membered ring structure in which one or more of the atoms in the ring is an element other than carbon, e.g., N, O or S. Heterocyclyl may be aromatic or non-aromatic, i.e., may be saturated, partially or fully unsaturated.

Preferred heterocyclyl groups include pyridyl, pyridazinyl, pyrimidinyl, isoquinolinyl, quinolinyl, quinazolinyl, imidazolyl, pyrrolyl, furanyl, thienyl, thiazolyl, benzothiazolyl, piperidinyl, pyrrolidinyl, tetrahydrofuranyl, tetrahydropyranyl, and morphonlinyl groups.

"Substituted heterocyclyl" means that the heterocyclyl group is substituted by one or more substituents wherein the substituents include alkoxy, alkylamino, aryl, carbalkoxy, carbamoyl, cyano, halo, heterocyclyl, trihalomethyl, hydroxy, mercaptyl, alkylmercaptyl or nitro.

"Hydroxyalkyl" means an alkyl group substituted by a hydroxy group. Hydroxy lower alkyl groups are preferred. Exemplary preferred groups include hydroxymethyl, 2-hydroxyethyl, 2-hydroxypropyl and 3-hydroxypropyl.

"Prodrug" means a compound which is rapidly transformed *in vivo* to yield the parent peptide compound, for example by hydrolysis in blood. "Pharmaceutically acceptable prodrug" means a compound which is, within the scope of sound medical judgement, suitable for pharmaceutical use in a patient without undue toxicity, irritation, allergic response, and the like, and effective for the intended use, including a pharmaceutically acceptable ester as well as a zwitterionic form, where possible, of the peptide compounds of the invention. Pharmaceutically acceptable prodrugs according to the invention are described in T. Higuchi and V. Stella, Pro-drugs as Novel Delivery Systems, Vol. 14 of the A.C.S. Symposium Series, and in Edward B. Roche, ed., Bioreversible Carriers in Drug Design, American Pharmaceutical Association and Pergamon Press, 1987, both of which are incorporated herein by reference.

"Solvate" means a physical association of a compound of this invention with one or more solvent molecules. This physical association involves varying degrees of ionic and covalent bonding, including hydrogen bonding. In certain instances the solvate will be capable of isolation, for example when one or more solvent molecules are incorporated in the crystal lattice of the crystalline solid. "Solvate" encompasses both solution-phase and isolable solvates. Representative solvates include ethanolates, methanolates, and the like. "Hydrate" is a solvate wherein the solvent molecule (s) is/are $H_2O$.

The compounds of Formula I contain chiral (asymmetric) centers. The invention includes the individual stereoisomers and mixtures thereof. The individual isomers are prepared or isolated by methods well known in the art or by methods described herein.

The compounds described herein may be used in the form of the free base, in the form of acid addition salts or as hydrates. All such forms are within the scope of the invention. Acid addition salts are simply a more convenient form for use. In practice, use of the salt form inherently amounts to use of the base form. The acids which may be used to prepare the acid addition salts include preferably those which produce, when combined with the free base, pharmaceutically acceptable salts, that is, salts whose anions are non-toxic to the recipient in pharmaceutical doses of the salts, so that the beneficial anti-hypertensive, cardioprotective, anti-ischemic, and antilipolytic effects produced by the free base are not vitiated by side effects ascribable to the anions. Although pharamaceutically acceptable salts of the compounds of the invention are preferred, all acid addition salts are useful as sources of the free base form, even if the particular salt, per se, is desired only as an intermediate product as, for example, when the salt is formed only for purposes of purification and identification, or when it is used as an intermediate in preparing a pharmaceutically acceptable salt by ion exchange procedures. Pharmaceutically acceptable salts within the scope of the invention are those derived from the following acids: mineral acids such as hydrochloric acid, sulfuric acid, phosphoric acid, and sulfamic acid; and organic acids such as acetic acid, citric acid, lactic acid, tartaric acid, malonic acid, methanesulfonic acid, fumaric acid, ethanesulfonic acid, benzenesulfonic acid, p-toluenesulfonic acid, cyclohexylsulfamic acid, quinic acid and the like. The corresponding acid addition salts comprise the following: hydrochloride, sulfate, phosphate, sulfamate, acetate, citrate, lactate, tartarate, methanesulfonate, fumarate, ethanesulfonate, benzenesulfonate, p-toluenesulfonate, cyclohexylsulfonate and quinate, respectively.

The acid addition salts of the compounds of the invention are conveniently prepared either by dissolving the free base in aqueous or aqueous-alcohol solution or other suitable solvents containing the appropriate acid and isolating the salt by evaporating the solution, or by reacting the free base and acid in an organic solvent, in which case the salt separates directly or can be obtained by concentration of the solution.

Included within the scope of Formula I are classes of compounds which may be characterized generally as N6-substituted adenosines; N6-substituted carbocyclic adenosines (or, alternatively, dihydroxy[N6-substituted-9-adenyl]cyclopentanes) and N-oxides thereof; and N6-substituted-N'-1-deazaaristeromycins (or, alternatively, dihydroxy [N7-substituted[4,5-b]imidazopyridyl]-cyclopentanes). Also within the scope of Formula I are the 5'-alkylcarboxamide derivatives of the adenosines, the carbocyclic adenosines and the 1-deazaaristeromycins, the derivatives of compounds of the above classes in which one or both of the 2- or 3- hydroxyl groups of the cyclopentane ring or, in the cases of classes of compounds containing the ribose moiety, the 2'- or 3'- hydroxyl groups of the ribose ring are substituted. Such derivatives may themselves comprise the biologically active chemical entity useful in the treatment of hypertension and myocardial ischemia, and as cardioprotective and antilipolytic agents, or may act as pro-drugs to such biologically active compounds which are formed therefrom under physiological conditions.

Representative compounds to use include: (2R,3R,4S,5R)-2-hydroxymethyl-5-[6-[1-(5-chloropyridin-2-yl)-pyrrolidin-3(S)-ylamino]-purin-9-yl]-tetrahydrofuran-3,4-diol,)-(2R,3S,4R,5R)-2-hydroxymethyl-5-[6-[1-(5-trifluoromethylpyridin-2-yl)-pyrrolidin-3(R)-ylamino]-purin-9-yl]tetrahydrofuran-3,4-diol, (2R,3R,4S,5R)-2-hydroxymethyl-5-[6-[1-(5-trifluoromethylpyridin-2-yl)-pyrrolidin-3(S)-ylamino]-purin-9-yl]tetrahydrofuran-3,4-diol, (2R,3R,4S,5R)-2-hydroxymethyl-5-[6-[1-(4-trifluoromethylpyridin-2-yl)-pyrrolidin-3(S)-ylamino]-purin-9-yl]tetrahydrofuran-3,4-diol, (2R,3R,4S,5R) 2-hydroxymethyl-5-[6-[1-(5-bromopyridin-2-yl)-pyrrolidin-3(S)-ylamino]-purin-9-yl]-tetrahydrofuran-3,4-diol, (2R,3R,4S,5R)-2-hydroxymethyl-5-(6-(1-(4-nitrophenyl)-pyrrolidin-3(S)-ylamino)-purin-9-yl) tetrahydrofuran-3,4-diol, (2R,3R,4S,5R)-2-hydroxymethyl-5-[6-(5'-trifluoromethyl-3,4,5,6-tetrahydro-2H-[1,2']-bipyridinyl-3-yl)-purin-9-yl]tetrahydrofuran-3,4-diol, (2R,3R,4S,5R)-2-hydroxymethyl-5-[6-(phenylpyrrolidin-3(S)-ylamino)-purin-9-yl]tetrahydrofuran-3,4-diol, (2R,3R,4S,5R)-2-hydroxymethyl-5-[6-(1-pyridin-2-ylpyrrolidin-3(S)-ylamino]-purin-9-yl]tetrahydrofuran-3,4-diol, (2R,3R,4S,5R)-2-hydroxymethyl-5-[6-[1-(4-chlorophenyl)-pyrrolidin-3(S)-ylamino]-purin-9-yl]-tetrahydrofuran-3,4-diol, (2R,3R,4S,5R)-2-hydroxymethyl-5-[6-[1-(5-methylpyridin-2-yl)-pyrrolidin-3(S)-ylamino]-purin-9-yl]tetrahydrofuran-3,4-diol, (2R,3R,4S,5R)-2-hydroxymethyl-5-[6-[1-(5-thiophen-2-ylpyridin-2-yl)-pyrrolidin-3(S)-ylamino]-purin-9-yl]tetrahydrofuran-3,4-diol, (2R,3R,4S,5R)-2-hydroxymethyl-5-[6-[1-(5-methylmercaptopyridin-2-yl)-pyrrolidin-3(S)-ylamino]-purin-9-yl]tetrahydrofuran-3,4-diol, (2R,3R,4S,5R)-2-hydroxymethyl-5-[6-[1-(6-methoxypyrimidin-4-yl)pyrrolidin-3 (S)-ylamino]-purin-9-yl]tetrahydrofuran-3,4-diol, (2R,3R,4S,5R)-2-hydroxymethyl -5-[6-[1-(6-chloropyrimidin-4-yl)pyrrolidin-3(S)-ylamino]-purin-9-yl]-tetrahydrofuran-3,4-diol, (2R,3R,4S,5R)-2-hydroxymethyl-5-[6-[1-(6-chloropyridazin-3-yl)pyrrolidin-3-ylamino]-purin-9-yl]-tetrahydrofuran-3,4-diol, (2R,3R,4S,5R)-2-methoxymethyl-5-[6-[1-(5-trifluoromethylpyridin-2-yl)-pyrrolidin-3(S)-ylamino]-purin-9-yl]tetrahydrofuran-3,4-diol, (1S,2R,3S,4R)-2,3-dihydroxy-4-[6-[1-(5-trifluormethylpyridin-2-yl)pyrrolidin-3-ylamino]-purin-9-yl]cyclopentanecarboxylic acid ethylamide, (1S,2R,3R,5R)-3-hydroxymethyl-5-[6-[1-(4-nitrophenyl)piperidin-4-yl]-purin-9-yl]cyclopentane-1,2-diol, (1S,2R,3R,5R)-3-hydroxymethyl-5-[6-((3S)-pyrrolidin-3-ylamino)-purin-9-yl] cyclopentane-1,2-diol dihydrochloride, (1S,2R,3R,5R)-3-hydroxymethyl-5-[6-[1-(4-nitrophenyl)pyrrolidin-3-ylamino]-purin-9-yl]cyclopentane-1,2-diol, (1S,2R,3R,5R)-3-hydroxymethyl-5-[6-[1-(5-trifluoromethylpyridin-2-yl)pyrrolidin-3(R)-ylamino]-purin-9-yl]cyclopentane-1,2-diol, (1S,2R,3R,5R)-3-hydroxymethyl-5-[6-((3R)-pyrrolidin-3-ylamino)-purin-9-yl] cyclopentane-1,2-diol, (1S,2R3R,5R)-3-hydroxymethyl-5-[6-[1-(5-trifluoromethylpyridin-2-yl)pyrrolidin-3(S)-ylamino]-purin-9-yl]cyclopentane-1,2-diol, 4(R)-1-benzyl-4-[9-(2,3-dihydroxy-4-hydroxymethylcyclopentyl)-9H-purin-6-ylamino] pyrrolidin-2-one hydrochloride, (1R,2S,3R,5S)-5-methyl-3-[6-[1-(5-trifluoromethylpyridin-2-yl)pyrrolidin-3(S)-ylamino]-purin-9-yl]cyclopentane-1,2-diol, (1S,2R3R,5R)-5-[6-[1-(5-bromopyridin-2-yl)pyrrolidin-3(S)-ylamino]-purin-9-yl]-3-hydroxymethylcyclopentane-1,2-diol, (1S,2R,3R,5R)-5-[6-[1-(5-chloropyridin-2-yl)pyrrolidin-3(S)-ylamino]-purin-9-yl]-3-hydroxymethylcyclopentane-1,2-diol, (1S,2R,3R,5R)-3-hydroxymethyl-5-[6-[1-(4-trifluoromethylpyridin-2-yl)pyrrolidin-3(S)-ylamino]-purin-9-yl]cyclopentane-1,2-diol, (1S,2R,3R,5R)-3-hydroxymethyl-5-[6-[1-(pyridin-2-yl)pyrrolidin-3(S)-ylamino]-purin-9-yl]cyclopentane-1,2-diol, 4(S)-1-benzyl-4-[9-(2,3-dihydroxy-4-hydroxymethylcyclopentyl)-9H-purin-6-ylamino]pyrrolidin-2-one hydrochloride, (1S,2R,3R,5R)-3-hydroxymethyl-5-[6-[1-(quinolin-3-yl)pyrrolidin-3(S)-ylamino]-purin-9-yl]cyclopentane-1,2-diol, (1S,2R,3R,5R)-3-hydroxymethyl-5-[6-[1-S-(4-nitrophenyl)-pyrrolidin-3(S)-ylamino]-purin-9-yl]cyclopentane-1,2-diol, (1S,2R,3R,5R)-5-[6-[1-(4,5-bistrifluorpyridin-2-yl)pyrrolidin-3(S)-ylamino]-purin-9-yl]-3-hydroxymethylcyclopentane-1,2-diol, (1S,2R,3R,5R)-3-methoxymethyl-5-[6-[1-(5-trifluoromethylpyridin2-yl)pyrrolidin-3(S)-ylamino]-purin-9-yl]cyclopentane-1,2-diol, (1S,2R,3R,5R)-3-hydroxymethyl-5-[6-[1-(phenyl)-pyrrolidin-3(S)-ylamino]-purin-9-yl]cyclopentane-1,2-diol, 4-[3(S)-[9-(2,3-dihydroxy-4-hydroxymethylcyclopentyl)-9H-purin-6-ylamino]pyrrolidin-1-yl]benzonitrile, (1S,2R,3R,5R)-3-hydroxymethyl-5-[6-[1-(isoquinolin-1-yl)pyrrolidin-3(S)-ylamino]-purin-9-yl]cyclopentane-1,2-diol, (1S,2R,3R,5R)-5-[6-[1-(6-bromoquinolin-2-yl)pyrrolidin-3(S)-ylamino]-purin-9-yl]-3-hydroxymethylcyclopentane-1,2- diol, (1S,2R,3R,5R)-5-[6-[1-(4-chlorophenyl)pyrrolidin-3(S)-ylamino]-purin-9-yl]-3-hydroxymethylcyclopentane-1,2-diol, (1S,2R,3R,5R)-3-[6-[1-(3-chloro-5-trifluoromethylpyridin-2-yl)pyrrolidin-3(S)-ylamino]-purin-9-yl]-3-methoxymethylcyclopentane-1,2-diol, (1S,2R,3R,5R)-3-isopropoxymethyl-5-[6-[1-(5-trifluoromethylpyridin2-yl)pyrrolidin-3(S)-ylamino]-purin-9-yl]cyclopentane-1,2-diol, (1S,2R,3R,5R)-3-isopropoxymethyl-5-[6-[1-(4-trifluoromethylpyridin2-yl)pyrrolidin-3(S)-ylamino]-purin-9-yl]cyclopentane-1,2-diol, (1S,2R,3R,5R)-5-[6-[1-(6-chloropyrimidin-4-yl)pyrrolidin-3(S)-ylamino]-purin-9-yl]-3-hydroxymethylcyclopentane-1,2-diol, (1S,2R,3R,5R)-5-[6-[1-(6-chloropyrimidin-4-yl)pyrrolidin-3(S)-ylamino]-purin-9-yl]-3-hydroxymethylcyclopentane-1,2-diol, (1S,2R,3R,5R)-5-[6-[1-(6-chloropyrimidin-4-yl)pyrrolidin-3(S)-ylamino]-purin-9-yl]-3-methoxymethylcyclopentane-1,2-diol, (1S,2R,3R,5R)-5-[6-[1-(6-chloropyridazin-3-yl)pyrrolidin-3(S)-ylamino]-purin-9-yl]-3-hydroxymethylcyclopentane-1,2-diol, (1S,2R,3R,5R)-3-methoxymethyl-5-[6-[1-(6-methoxypyrimidin-4-yl)pyrrolidin-3(S)-ylamino]-purin-9-yl]cyclopentane-1,2-diol, (1S,2R,3R,5R)-5-[6-[1-(6-chloropyridazin-3-yl)pyrrolidin-3(S)-ylamino]-purin-9-yl]-3-methoxymethylcyclopentane-1,2-diol, (1S,2R,3R,5R)-5-(-[6-[1-(4-trifluoromethylphenyl)-pyrrolidin-3(S)-ylamino]-purin-9-yl]-3-hydroxymethylcyclopentane-1,2-diol, (1S,2R,3R,5R)-5-[6-[1-(5-bromopyridin-2-yl)pyrrolidin-3(S)-ylamino]-purin-9-yl]-3-methoxymethylcyclopentane-1,2- diol, (1 S,2R,3R,5R)-5-[6-[1-(5-chlorpyridin-2-yl)pyrrolidin-3(S)-ylamino]-purin-9-yl]-3-methoxymethylcyclopentane-1,2-diol, (1 S,2R,3R,5R)-3-methoxymethyl-5-[6-[1-(4-trifluoromethylphenyl)pyrrolidin-3(S)-ylamino]-purin-9-yl]cyclopentane-1,2-diol, (1S,2R,3R,5R)-5-(-[6-[1-(4-chlorophenyl)-pyrrolidin-3(S)-ylamino]-purin-9-yl]-3-methoxymethylcyclopentane-1,2-diol, (1S,2R,3R,5R)-5-[6-[1-(3-chlorophenyl)-pyrrolidin-3(S)-ylamino]-purin-9-yl]-3-methoxymethylcyclopentane-1,2-diol, (1S,2R,3R,5R)-5-[6-[1-(3-chlorophenyl)-pyrrolidin-3(S)-ylamino]-purin-9-yl]-3-hydroxymethylcyclopentane-1,2-diol, (1S,2R,3R,5R)-3-methoxymethyl-5-[6-[1-phenylpyrrolidin-3-(S)-ylamino]-purin-9-yl]cyclopentan-1,2-diol, (1S,2R,3R,5R)-3-[6-(1-benzyl-pyrrolidin-3(S)-ylamino)purin-9-yl]5-hydroxymethylcyclopentane-1,2-diol, (1S,2R,3R,5R)-3-[6-(1-benzyl-pyrrolidin-3(S)-ylamino)purin-9-yl]5-methoxymethylcyclopentane-1,2-diol, (1S,2R,3S,4R)-2,3-dihydroxy-4-{6-[1-(5-trifluoromethyl-pyridin-2-yl)-pyrrolidin-3(S)-ylamino]-purin-9-yl}-cyclopentanecarboxylic acid-1 (S)-methylpropylamide, and (1S,2R,3S,4R)-2,3-dihydroxy-4-{6-[1-(5-trifluoromethyl-pyridin-2-yl)-pyrrolidin-3(S)-ylamino]-purin-9-yl}-cyclopentanecarboxylic acid-1(R)-methylpropylamide.

A preferred class of compounds to use is described by Formula I wherein K is N, T is hydroxymethyl or methoxymethyl, A and B are hydroxy, X is

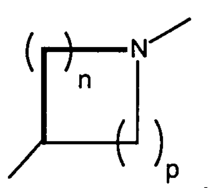

and n + p is 3 or 4, or pharmaceutically acceptable salts thereof. Representative compounds of this preferred class of compounds include (2R,3R,4S,5R)-2-hydroxymethyl-5-[6-[1-(5-chloropyridin-2-yl)-pyrrolidin-3(S)-ylamino]-purin-9-yl]-tetrahydrofuran-3,4-diol, (2R,35,4R,5R)-2-hydroxymethyl-5-[6-[1-(5-trifluoromethylpyridin-2-yl)-pyrrolidin-3(R)-ylamino]-purin-9-yl]tetrahydrofuran-3,4-diol, (2R,3R,4S,5R)-2-hydroxymethyl-5-[6-[1-(5-trifluoromethylpyridin-2-yl)-pyrrolidin-3(S)-ylamino]-purin-9-yl]tetrahydrofuran-3,4-diol,(2R,3R,4S,5R)-2-hydroxymethyl-5-[6-[1-(4-trifluoromethylpyridin-2-yl)-pyrrolidin-3(S)-ylamino]-purin-9-yl]tetrahydrofuran-3,4-diol, (2R,3R,4S,5R) 2-hydroxymethyl-5-[6-[1-(5-bromopyridin-2-yl)-pyrrolidin-3(S)-ylamino]-purin-9-yl]-tetrahydrofuran-3,4-diol, (2R,3R,4S,5R)-2-hydroxymethyl-5-(6-(1-(4-nitrophenyl)-pyrrolidin-3(S)-ylamino)-purin-9-yl) tetrahydrofuran-3,4-diol, (2R,3R,4S,5R)-2-hydroxymethyl-5-[6-(5'-trifluoromethyl-3,4,5,6-tetrahydro-2H-[1,2']-bipyridinyl-3-yl)-purin-9-yl]tetrahydrofuran-3,4-diol, (2R,3R,4S,5R)-2-hydroxymethyl-5-[6-(phenylpyrrolidin-3(S)-ylamino)-purin-9-yl]tetrahydrofuran-3,4-diol, (2R,3R,4S,5R)-2-hydroxymethyl-5-[6-(1-pyridin-2-ylpyrrolidin-3(S)-ylamino]-purin-9-yl]tetrahydrofuran-3,4-diol, (2R,3R,4S,5R)-2-hydroxymethyl-5-[6-[1-(4-chlorophenyl)-pyrrolidin-3(S)-ylamino]-purin-9-yl]-tetrahydrofuran-3,4-diol, (2R,3R,4S,5R)-2-hydroxymethyl-5-[6-[1-(5-methylpyridin-2-yl)-pyrrolidin-3(S)-ylamino]-purin-9-yl]tetrahydrofuran-3,4-diol, (2R,3R,4S,5R)-2-hydroxymethyl-5-[6-[1-(5-thiophen-2-ylpyridin-2-yl)-pyrrolidin-3(S)-ylamino]-purin-9-yl]tetrahydrofuran-3,4-diol, (2R,3R,4S,5R)-2-hydroxymethyl-5-[6-[1-(5-methylmercaptopyridin-2-yl)-pyrrolidin-3(S)-ylamino]-purin-9-yl] tetrahydrofuran-3,4-diol, (2R,3R,4S,5R)-2-hydroxymethyl-5-[6-[1-(6-methoxypyrimidin-4-yl)pyrrolidin-3(S)-ylamino]-purin-9-yl]tetrahydrofuran-3,4-diol, (2R,3R,4S,5R)-2-hydroxymethyl-5-[6-[1-(6-chloropyrimidin-4-yl)pyrrolidin-3(S)-ylamino]-purin-9-yl]-tetrahydrofuran-3,4-diol, (2R,3R,4S,5R)-2-hydroxymethyl-5-[6-[1-(6-chloropyridazin-3-yl)pyrrolidin-3-ylamino]-purin-9-yl]-tetrahydrofuran-3,4-diol, (2R,3R,4S,5R)-2-methoxymethyl-5-[6-[1-(5-trifluoromethylpyridin-2-yl)-pyrrolidin-3(S)-ylamino]-purin-9-yl]tetrahydrofuran-3,4-diol, (1S,2R,3R,5R)-3-hydroxymethyl-5-[6-[1-(4-nitrophenyl)piperidin-4-yl]-purin-9-yl]cyclopentane-1,2-diol, (1S,2R,3R,5R)-3-hydroxymethyl-5-[6-((3S)-pyrrolidin-3-ylamino)-purin-9-yl] cyclopentane-1,2-diol dihydrochloride, (1S,2R,3R,5R)-3-hydroxymethyl-5-[6-[1-(4-nitrophenyl)pyrrolidin-3-ylamino]-purin-9-yl]cyclopentane-1,2-diol, (1S,2R,3R,5R)-3-hydroxymethyl-5-[6-[1-(5-trifluoromethylpyridin-2-yl) pyrrolidin-3(R)-ylamino]-purin-9-yl]cyclopentane-1,2-diol, (1S,2R,3R,5R)-3-hydroxymethyl-5-[6-((3R)-pyrrolidin-3-ylamino)-purin-9-yl] cyclopentane-1,2-diol, (1S,2R,3R,5R)-3-hydroxymethyl-5-[6-[1-(5-trifluoromethylpyridin-2-yl) pyrrolidin-3(S)-ylamino]-purin-9-yl]cyclopentane-1,2-diol, (1S,2R,3R,5R)-5-[6-[1-(5-bromopyridin-2-yl)pyrrolidin-3(S)-ylamino]-purin-9-yl]-3-hydroxymethylcyclopentane-1,2-diol, (1S,2R,3R,5R)-5-[6-[1-(5-chloropyridin-2-yl)pyrrolidin-3 (S)-ylamino]-purin-9-yl]-3-hydroxymethylcyclopentane-1,2-diol, (1S,2R,3R,5R)-3-hydroxymethyl-5-[6-[1-(4-trifluoromethylpyridin-2-yl)pyrrolidin-3(S)-ylamino]-purin-9-yl]cyclopentane-1,2-diol, (1S,2R,3R,5R)-3-hydroxymethyl-5-[6-[1-(pyridin-2-yl)pyrrolidin-3(S)-ylamino]-purin-9-yl]cyclopentane-1,2-diol,(1S,2R,3R,5R)-3-hydroxymethyl-5-[6-[1-(quinolin-3-yl)pyrrolidin-3(S)-ylamino]-purin-9-yl]cyclopentane-1,2-diol,(1S,2R,3R,5R)-3-hydroxymethyl-5-[6-[1-S-(4-nitrophenyl)-pyrrolidin-3(S)-ylamino]-purin-9-yl]cyclopentane-1,2-diol, (1S,2R,3R,5R)-5-[6-[1-(4,5-bistrifluorpyridin-2-yl)pyrrolidin-3(S)-ylamino]-purin-9-yl]-3-hydroxymethylcyclopentane-1,2-diol, (1S,2R,3R,5R)-3-methoxymethyl-5-[6-[1-(5-trifluoromethylpyridin2-yl)pyrrolidin-3(S)-ylamino]-purin-9-yl]cyclopentane-1,2-diol, (1S,2R,3R,5R)-3-hydroxymethyl-5-[6-[1-(phenyl)-pyrrolidin-3(S)-ylamino]-purin-9-yl]cyclopentane-1,2-diol, 4-[3(S)-[9-(2,3-dihydroxy-4-hydroxymethylcyclopentyl)-9H-purin-6-ylamino]pyrrolidin-1-yl]benzonitrile, (1S,2R,3R,5R)-3-hydroxymethyl-5-[6-[1-(isoquinolin-1-yl)pyrrolidin-3(S)-ylamino]-purin-9-yl]cyclopentane-1,2-diol, (1S,2R,3R,5R)-5-[6-[1-(6-bromoquinolin-2-yl)pyrrolidin-3(S)-ylamino]-purin-9-yl]-3-hydroxymethylcyclopentane-1,2-diol, (1S,2R,3R,5R)-5-[6-[1-(4-chlorophenyl)pyrrolidin-3 (S)-ylamino]-purin-9-yl]-3-hydroxymethylcyclopentane-1,2-diol, (1S,2R,3R,5R)-3-[6-[1-(3-chloro-5-trifluoromethylpyridin-2-yl)pyrrolidin-3(S)-ylamino]-purin-9-yl]-3-methoxymethylcyclopentane-1,2-diol,(1S,2R,3R,5R)-5-[6-[1-(6-chloropyrimidin-4-yl)pyrrolidin-3(S)-ylamino]-purin-9-yl]-3-hydroxymethylcyclopentane-1,2-diol,(1S,2R,3R,5R)-5-[6-[1-(6-chloropyrimidin-4-yl)pyrrolidin-3(S)-ylamino]-purin-9-yl]-3-hydroxymethylcyclopentane-1,2-diol, (1S,2R,3R,5R)-5-[6-[1-(6-chloropyrimidin-4-yl)pyrrolidin-3(S)-ylamino]-purin-9-yl]-3-methoxymethylcyclopentane-1,2-diol, (1S,2R,3R,5R)-5-[6-[1-(6-chloropyridazin-3-yl)pyrrolidin-3(S)-ylamino]-purin-9-yl]-3-hydroxymethylcyclopentane-1,2-diol, (1S,2R,3R,5R)-3-methoxymethyl-5-[6-[1-(6-methoxypyrimidin-4-yl)pyrrolidin-3(S)-ylamino]-purin-9-yl]cyclopentane-1,2-diol, (1S,2R,3R,5R)-5-[6-[1-(6-chloropyridazin-3-yl)pyrrolidin-3(S)-ylamino]-purin-9-yl]-3-methoxymethylcyclopentane-1,2-diol, (1S,2R,3R,5R)-5-(-[6-[1-(4-trifluoromethylphenyl)-pyrrolidin-3(S)-ylamino]-purin-9-yl] -3-hydroxymethylcyclopentane-1,2-diol, (1S,2R,3R,5R)-5-[6-[1-(5-bromopyridin-2-yl)pyrrolidin-3(S)-ylamino]-purin-9-yl]-3-methoxymethylcyclopentane-1,2-diol, (1S,2R,3R,5R)-5-[6-[1-(5-chlorpyridin-2-yl)pyrrolidin-3(S)-ylamino]-purin-9-yl]-3-methoxymethylcyclopentane-1,2-diol, (1 S,2R,3R,5R)-3-methoxymethyl-5-[6-[1-(4-trifluoromethylphenyl)pyrrolidin-3(S)-ylamino]-purin-9-yl]cyclopentane-1,2-diol, (1S,2R,3R,5R)-5-(-[6-[1-(4-chlorophenyl)-pyrrolidin-3(S)-ylamino]-purin-9-yl]-3-methoxymethylcyclopentane-1,2-diol, (1S,2R,3R,5R)-5-[6-[1-(3-chlorophenyl)-pyrrolidin-3(S)-ylamino]-purin-9-yl]-3-methoxymethylcyclopentane-1,2-diol, (1S,2R,3R,5R)-5-[6-[1-(3-chlorophenyl)-pyrrolidin-3(S)-ylamino]-purin-9-yl]-3-hydroxymethylcyclopentane-1,2-diol, (1S,2R,3R,5R)-3-methoxymethyl-5-[6-[1-phenylpyrrolidin-3-(S)-ylamino]-purin-9-yl]cyclopentan-1,2-diol, (1S,2R,3R,5R)-3-[6-(1-benzyl-pyrrolidin-3(S)-ylamino)purin-9-yl]5-hydroxymethylcyclopentane-1,2-diol, and (1S,2R,3R,5R)-3-[6-(1-benzyl-pyrrolidin-3(S)-ylamino)purin-9-yl]5-methoxymethylcyclopentane-1,2-diol.

Another preferred class of compounds to use is described by Formula I wherein Q is $CH_2$, K is N, T is

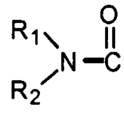

,

wherein $R_1$ is H and $R_2$ is lower alkyl, A and B are hydroxy, X is

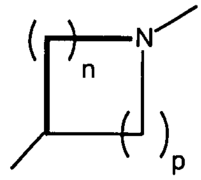

and n + p is 3 or 4, or pharmaceutically acceptable salts thereof. Representative compounds of this other preferred class of compounds include , (1S,2R,3S,4R)-2,3-dihydroxy-4-[6-[1-(5-trifluormethylpyridin-2-yl)pyrrolidin-3-ylamino]-purin-9-yl]cyclopentanecarboxylic acid ethylamide, (1 S,2R,3S,4R)-2,3-dihydroxy-4-{6-[1-(5-trifluoromethyl-pyridin-2-yl)-pyrrolidin-3(S)-ylamino]-purin-9-yl}-cyclopentanecarboxylic acid-1(S)-methylpropylamide, and (1S,2R,3S,4R)-2,3-dihydroxy-4-{6-[1-(5-trifluoromethylpyridin-2-yl)-pyrrolidin-3(S)-ylamino]-purin-9-yl}-cyclopentanecarboxylic acid-1(R)-methylpropylamide.

A more preferred class of compounds to use is described by Formula I wherein Q is $CH_2$, K is N, T is hydroxymethyl or methoxymethyl, A and B are hydroxy, X is

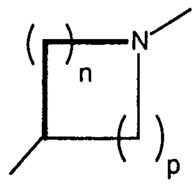

and n + p is 3 or 4, or pharmaceutically acceptable salts thereof. Representative compounds of this more preferred class of compounds include (1S,2R,3R,5R)-3-hydroxymethyl-5-[6-[1-(4-nitrophenyl)piperidin-4-yl]-purin-9-yl]cyclopentane-1,2-diol, (1S,2R,3R,5R)-3-hydroxymethyl-5-[6-((3S)-pyrrolidin-3-ylamino)-purin-9-yl] cyclopentane-1,2-diol dihydrochloride, (1S,2R,3R,5R)-3-hydroxymethyl-5-[6-[1-(4-nitrophenyl)pyrrolidin-3-ylamino]-purin-9-yl]cyclopentane-1,2-diol, (1S,2R,3R,5R)-3-hydroxymethyl-5-[6-[1-(5-trifluoromethylpyridin-2-yl)pyrrolidin-3(R)-ylamino]-purin-9-yl]cyclopentane-1,2-diol, (1S,2R,3R,5R)-3-hydroxymethyl-5-[6-((3R)-pyrrolidin-3-ylamino)-purin-9-yl] cyclopentane-1,2-diol, (1S,2R,3R,5R)-3-hydroxymethyl-5-[6-[1-(5-trifluoromethylpyridin-2-yl)pyrrolidin-3(S)-ylamino]-purin-9-yl]cyclopentane-1,2-diol, (1S,2R,3R,5R)-5-[6-[1-(5-bromopyridin-2-yl)pyrrolidin-3(S)-ylamino]-purin-9-yl]-3-hydroxymethylcyclopentane-1,2-diol, (1S,2R,3R,5R)-5-[6-[1-(5-chloropyridin-2-yl)pyrrolidin-3(S)-ylamino]-purin-9-yl]-3-hydroxymethylcyclopentane-1,2-diol, (1S,2R,3R,5R)-3-hydroxymethyl-5-[6-[1-(4-trifluoromethylpyridin-2-yl)pyrrolidin-3(S)-ylamino]-purin-9-yl]cyclopentane-1,2-diol, (1S,2R,3R,5R)-3-hydroxymethyl-5-[6-[1-(pyridin-2-yl)pyrrolidin-3(S)-ylamino]-purin-9-yl]cyclopentane-1,2-diol, (1S,2R,3R,5R)-3-hydroxymethyl-5-[6-[1-(quinolin-3-yl)pyrrolidin-3(S)-ylamino]-purin-9-yl]cyclopentane-1,2-diol, (1S,2R,3R,5R)-3-hydroxymethyl-5-[6-[1-S-(4-nitrophenyl)-pyrrolidin-3(S)-ylamino]-purin-9-yl]cyclopentane-1,2-diol, (1S,2R,3R,5R)-5-[6-[1-(4,5-bistrifluorpyridin-2-yl)pyrrolidin-3(S)-ylamino]-purin-9-yl]-3-hydroxymethylcyclopentane-1,2-diol, (1S,2R,3R,5R)-3-methoxymethyl-5-[6-[1-(5-trifluoromethylpyridin2-yl)pyrrolidin-3(S)-ylamino]-purin-9-yl]cyclopentane-1,2-diol, (1S,2R,3R,5R)-3-hydroxymethyl-5-[6-[1-(phenyl)-pyrrolidin-3(S)-ylamino]-purin-9-yl]cyclopentane-1,2-diol, 4-[3(S)-[9-(2,3-dihydroxy-4-hydroxymethylcyclopentyl)-9H-purin-6-ylamino]pyrrolidin-1-yl]benzonitrile, (1S,2R,3R,5R)-3-hydroxymethyl-5-[6-[1-(isoquinolin-1-yl)pyrrolidin-3(S)-ylamino]-purin-9-yl]cyclopentane-1,2-diol, (1S,2R,3R,5R)-5-[6-[1-(6-bromoquinolin-2-yl)pyrrolidin-3(S)-ylamino]-purin-9-yl]-3-hydroxymethylcyclopentane-1,2-diol, (1S,2R,3R,5R)-5-[6-[1-(4-chlorophenyl)pyrrolidin-3(S)-ylamino]-purin-9-yl]-3-hydroxymethylcyclopentane-1,2-diol, (1S,2R,3R,5R)-3-[6-[1-(3-chloro-5-trifluoromethylpyridin-2-yl)pyrrolidin-3(S)-ylamino]-purin-9-yl]-3-methoxymethylcyclopentane-1,2-diol, (1S,2R,3R,5R)-5-[6-[1-(6-chloropyrimidin-4-yl)pyrrolidin-3(S)-ylamino]-purin-9-yl]-3-hydroxymethylcyclopentane-1,2-diol, (1S,2R,3R,5R)-5-[6-[1-(6-chloropyrimidin-4-yl)pyrrolidin-3(S)-ylamino]-purin-9-yl]-3-hydroxymethylcyclopentane-1,2-diol(1S,2R,3R,5R)-5-[6-[1-(6-chloropyrimidin-4-yl)pyrrolidin-3(S)-ylamino]-purin-9-yl]-3-methoxymethylcyclopentane-1,2-diol(1S,2R,3R,5R)-5-[6-[1-(6-chloropyridazin-3-yl)pyrrolidin-3(S)-ylamino]-purin-9-yl]-3-hydroxymethylcyclopentane-1,2-diol, (1S,2R,3R,5R)-3-methoxymethyl-5-[6-[1-(6-methoxypyrimidin-4-yl)pyrrolidin-3(S)-ylamino]-purin-9-yl]cyclopentane-1,2-diol, (1S,2R,3R,5R)-5-[6-[1-(6-chloropyridazin-3-yl)pyrrolidin-3(S)-ylamino]-purin-9-yl]-3-methoxymethylcyclopentane-1,2-diol, (1S,2R,3R,5R)-5-(-[6-[1-(4-trifluoromethylphenyl)-pyrrolidin-3(S)-ylamino]-purin-9-yl] -3-hydroxymethylcyclopentane-1,2-diol, (1S,2R,3R,5R)-5-[6-[1-(5-bromopyridin-2-yl)pyrrolidin-3(S)-ylamino]-purin-9-yl]-3-methoxymethylcyclopentane-1,2- diol, (1S,2R,3R,5R)-5-[6-[1-(5-chlorpyridin-2-yl)pyrrolidin-3(S)-ylamino]-purin-9-yl]-3-methoxymethylcyclopentane-1,2-diol, (1

S,2R,3R,5R)-3-methoxymethyl-5-[6-[1-(4-trifluoromethylphenyl)pyrrolidin-3(S)-ylamino]-purin-9-yl]cyclopentane-1,2-diol, (1S,2R,3R,5R)-5-(-[6-[1-(4-chlorophenyl)-pyrrolidin-3(S)-ylamino]-purin-9-yl]-3-methoxymethylcyclopentane-1,2-diol, (1S,2R,3R,5R)-5-[6-[1-(3-chlorophenyl)-pyrrolidin-3(S)-ylamino]-purin-9-yl]-3-methoxymethylcyclopentane-1,2-diol, (1S,2R,3R,5R)-5-[6-[1-(3-chlorophenyl)-pyrrolidin-3(S)-ylamino]-purin-9-yl]-3-hydroxymethylcyclopentane-1,2-diol,(1S,2R,3R,5R)-3-methoxymethyl-5-[6-[1-phenylpyrrolidin-3-(S)-ylamino]-purin-9-yl]cyclopentan-1,2-diol, (1S,2R,3R,5R)-3-[6-(1-benzyl-pyrrolidin-3(S)-ylamino)purin-9-yl]5-hydroxymethylcyclopentane-1,2-diol, and (1S,2R,3R,5R)-3-[6-(1-benzyl-pyrrolidin-3(S)-ylamino)purin-9-yl]5-methoxymethylcyclopentane-1,2-diol.

Most preferred compound to use in the present invention include (1S,2R,3R,5R)-3-hydroxymethyl-5-[6-[1-(5-trifluoromethylpyridin-2-yl)pyrrolidin-3(S)-ylamino]-purin-9-yl]cyclopentane-1,2-diol, (1S,2R,3R,5R)-3-hydroxymethyl-5-[6-[1-(4-trifluoromethylpyridin-2-yl)pyrrolidin-3(S)-ylamino]-purin-9-yl]cyclopentane-1,2-diol, (1S,2R,3R,5R)-3-methoxymethyl-5-[6-[1-(5-trifluoromethylpyridin-2-yl)pyrrolidin-3(S)-ylamino]-purin-9-yl]cyclopentane-1,2-diol and (1S,2R,3R,5R)-3-methoxymethyl-5-[6-[1-(4-trifluoromethylpyridin-2-yl)pyrrolidin-3(S)-ylamino]-purin-9-yl]cyclopentane-1,2-diol.

The amount of a compound of formula (I) which is necessary to achieve the desired biological effect depends on a number of factors, for example the specific compound chosen, the intended use, the mode of administration and the clinical condition of the patient. In general, the daily dose is in the range from 0.3 mg to 100 mg (typically from 3 mg to 50 mg) per day and per kilogram of body weight, for example 3-10 mg/kg/day. An intravenous dose may be, for example, in the range from 0.3 mg to 1.0 mg/kg, which can most suitably be administered as infusion of from 10 ng to 100 ng per kilogram and per minute. Suitable infusion solutions for these purposes may contain, for example, from 0.1 ng to 10 mg, typically from 1 ng to 10 mg, per milliliter. Single doses may contain, for example, from 1 mg to 10 g of the active ingredient. It is thus possible for ampoules for injections to contain, for example, from 1 mg to 100 mg, and single-dose formulations which can be administered orally, such as, for example, tablets or capsules, to contain, for example, from 1.0 to 1000 mg, typically from 10 to 600 mg. In the case of pharmaceutically acceptable salts, the aforementioned weight data are based on the weight of the salt of the compound of formula (I). For the prophylaxis or therapy of the above-mentioned conditions, the compounds of formula (I) can be used as the compound itself, but they are preferably in the form of a pharmaceutical composition with an acceptable carrier. The carrier must, of course, be acceptable in the sense that it is compatible with the other ingredients of the composition and is not hazardous for the patient's health. The carrier may be a solid or a liquid or both and is preferably formulated with the compound as single dose, for example as tablet which may contain from 0.05% to 95% by weight of the active ingredient. Further pharmaceutically active substances may likewise be present, including other compounds of formula (I). The pharmaceutical compositions according to the invention can be produced by one of the known pharmaceutical methods which essentially consist of mixing the ingredients with pharmacologically acceptable carriers and/or excipients.

Pharmaceutical compositions according to the invention are those suitable for oral, rectal, topical, peroral (for example sublingual) and parenteral (for example subcutaneous, intramuscular, intradermal or intravenous) administration although the most suitable mode of administration in each individual case depends on the nature and severity of the condition to be treated and on the nature of the compound of formula (I) used in each case. Coated formulations and coated slow-release formulations also lie within the scope of the invention. Formulations resistant to acid and gastric fluid are preferred. Suitable coatings resistant to gastric fluid comprise cellulose acetate phthalate, polyvinyl acetate, hydroxypropylmethylcellulose phthalate and anionic polymers of methacrylic acid and methyl methacrylate.

Suitable pharmaceutical compounds for oral administration may be in the form of separate units such as, for example, capsules, cachets, suckable tablets or tablets, each of which contain a defined amount of the compound of formula (I); as powders or granules; as solution or suspension in an aqueous or nonaqueous liquid; or as an oil-in-water or water-in-oil emulsion. These compositions may, as already mentioned, be prepared by any suitable pharmaceutical method which includes a step in which the active ingredient and the carrier (which may consist of one or more additional ingredients) are brought into contact. In general, the compositions are produced by uniform and homogeneous mixing of the active ingredient with a liquid and/or finely divided solid carrier, after which the product is shaped if necessary. Thus, for example, a tablet can be produced by compressing or molding a powder or granules of the compound, where appropriate with one or more additional ingredients. Compressed tablets can be produced by tabletting the compound in free-flowing form, such as, for example, a powder or granules, where appropriate mixed with a binder, lubricant, inert diluent and/or a (plurality of) surface-active/dispersing agent(s) in a suitable machine. Molded tablets can be produced by molding the compound which is in powder form and is moistened with an inert liquid diluent in a suitable machine.

Pharmaceutical compositions suitable for peroral (sublingual) administration comprise suckable tablets which contain a compound of formula (I) with a flavoring, normally sucrose and gum arabic or tragacanth, and pastilles which comprise the compound in an inert base such as gelatin and glycerol or sucrose and gum arabric.

Suitable pharmaceutical compositions for parenteral administration comprise preferably sterile aqueous preparations of a compound of formula (I), which are preferably isotonic with the blood of the intended recipient. These preparations are preferably administered intravenously, although administration may also take place by subcutaneous,

intramuscular or intradermal injection. These preparations can preferably be produced by mixing the compound with water and making the resulting solution sterile and isotonic with blood. , Injectable compositions according to the invention generally contain from 0.1 to 5% by weight of the active compound.

Suitable pharmaceutical compositions for rectal administration are preferably in the form of single-dose suppositories. These can be produced by mixing a compound of formula (I) with one or more conventional solid carriers, for example cocoa butter, and shaping the resulting mixture.

Suitable pharmaceutical compositions for topical application to the skin are preferably in the form of ointment, cream, lotion, paste, spray, aerosol or oil. Carriers which can be used are petrolatum, lanolin, polyethylene glycols, alcohols and combinations of two or more of these substances. The active ingredient is generally present in a concentration of from 0.1 to 15% by weight of the composition, for example from 0.5 to 2%.

Transdermal administration is also possible. Suitable pharmaceutical compositions for transdermal uses can be in the form of single plasters which are suitable for long-term close contact with the patient's epidermis. Such plasters suitably contain the active ingredient in an optionally buffered aqueous solution, dissolved and/or dispersed in an adhesive or dispersed in a polymer. A suitable active igredient concentration is about 1% to 35%, preferably about 3% to 15%. As a special possibility, the active ingredient can be released as described, for example, in Pharmaceutical Research, 2(6): 318 (1986) by electrotransport or iontophoresis.

The following preparations serve to illustrate the invention without restricting it, however.

Example A

Soft gelatin capsules containing 100 mg of active ingredient per capsule:

| | per capsule |
|---|---|
| Active ingredient | 100 mg |
| Triglyceride mixture fractionated from coconut fat | 400 mg |
| Capsule contents | 500 mg |

Example B

Emulsion containing 60 mg of active ingredient per 5 ml:

| | per 100 ml emulsion |
|---|---|
| Active ingredient | 1.2 g |
| Neutral oil | q.s. |
| Sodium carboxymethylcellulose | 0.6 g |
| Polyoxyethylene stearate | q.s. |
| Glycerol, pure | 0.2 to 2.0 g |
| Flavoring | q.s. |
| Water (deionized or distilled) | ad 100 ml |

Example C

Rectal pharmaceutical form containing 40 mg of active ingredient per suppository:

| | per suppository |
|---|---|
| Active ingredient | 40 mg |
| Suppository base | ad 2 g |

Example D

Tablets containing 40 mg of active ingredient per tablet:

| | per tablet |
|---|---|
| Active ingredient | 40 mg |
| Lactose | 600 mg |
| Corn starch | 300 mg |
| Soluble starch | 20 mg |
| Magnesium stearate | 40 mg |
| | 1000 mg |

Example E

Coated tablets containing 50 mg of active ingredient per coated tablet:

| | per coated tablet |
|---|---|
| Active ingredient | 50 mg |
| Corn starch | 100 mg |
| Lactose | 60 mg |
| Sec. calcium phosphate | 30 mg |
| Soluble starch | 5 mg |
| Magnesium stearate | 10 mg |
| Colloidal silica | 5 mg |
| | 260 mg |

Example F

The following formulas are suitable for producing the contents of hard gelatin capsules:

| | | |
|---|---|---|
| a) | Active ingredient | 100 mg |
| | Corn starch | 300 mg |
| | | 400 mg |

| | | |
|---|---|---|
| b) | Active ingredient | 140 mg |
| | Lactose | 180 mg |
| | Corn starch | 180 mg |
| | | 500 mg |

Example G

Drops can be produced in accordance with the following formula (100 mg of active ingredient in 1 ml = 20 drops):

| | |
|---|---|
| Active ingredient | 10 g |
| Methyl benzoate | 0.07 g |
| Ethyl benzoate | 0.03 g |
| Ethanol 96% pure | 5 ml |
| Demineralized water | ad 100 ml |

Compounds of this invention may be prepared by known methods or in accordance with the reaction sequences described in EP- A 0 912 520 (HOE 1996/S 013).

Following synthesis, compounds of the invention are typically purified by medium pressure liquid chromatography (MPLC), on a chromatotron, radially accelerated thin layer chromatography, flash chromatography or column chromatography through a silica gel or Florisil matrix, followed by crystallization. For compounds of Formula I wherein K is N, Q is O and T is $R_3O$-$CH_2$, typical solvent systems include chloroform:methanol, ethyl acetate:hexane, and

methylene chloride:methanol. Eluates may be crystallized from methanol, ethanol, ethyl acetate, hexane or chloroform, etc.

For compounds of Formula I, wherein K is N, Q is O, and T is $R_1R_2N$-C=O, typical solvent systems include chloroform:methanol. For example, eluates may be crystallized from 50-100% ethanol (aqueous).

For compounds of Formula I, wherein Q is $CH_2$, K is N or CH, and T is $R_1R_2N$-C=O, typical solvent systems include methylene chloride:methanol. For example, eluates may be crystallized from ethyl acetate with or without methanol, ethanol or hexane.

Compounds requiring neutralization may be neutralized with a mild base such as sodium bicarbonate, followed by washing with methylene chloride and brine. Products which are purified as oils are sometimes triturated with hexane/ethanol prior to final crystallization.

The method of the present invention is further illustrated and explained by the following Examples.

## EXAMPLE 1

Preparation of 5'-N-Ethyl-2',3'-isopropylidene-$N^6$-chloroadenosine-5'-uronamide

Step 1: $N^6$-Chloro-2',3'-isopropylideneadenosine

6-Chloropurine riboside (31.5 g), triethylorthoformate (73 mL) and TsOH (19.8 g) are stirred in 600 mL acetone for 2 hours at RT. The reaction mixture is concentrated in vacuo, combined with ethyl acetate and washed with saturated $NaHCO_3$ solution, and brine, dried ($Na_2SO_4$) and concentrated to yield $N^6$-Chloro-2',3'-isopropylideneadenosine as a white solid.

Step 2: $N^6$-Chloro-2',3'-Isopropylideneadenosine-5'-carboxylic acid

$N^6$-Chloro-2',3'-isopropylideneadenosine (4.5 g, 13.8 mmol) and 4-hydroxy-2,2,6,6-tetramethylpiperidinyloxy benzoate (4-hydroxy-TEMPO benzoate) (0.0381 g, 0.14 mmol) are combined in acetonitrile, 5% $NaHCO_3$ (87%) is added to the reaction mixture and sodium bromite hydrate (10.41 g, 55.1 mmol) is added portionwise at 0-5 °C. The reaction mixture is then allowed to warm to room temperature, and the solution was stirred vigorously for about 3 hours. 10 % tartaric acid solution is added and the aqueous layer is separated and extracted with ethyl acetate (3x). The combined organic layers are washed with 5% sodium bicarbonate solution (3x). The basic layers are combined and reacidified to pH 3 with concentrated hydrochloric acid. The aqueous layers are extracted with ethyl acetate (3x). The combined organic layers are then washed with brine and dried over magnesium sulfate. The filtrate is concentrated to an amorphous white solid, co-evaporated with 3 portions of toluene and dried in vacuo to give $N^6$-chloro-2',3'-isopropylideneadenosine-5'-carboxylic acid.

Step 3: 5'-N-Ethyl-2',3'-isopropylidene-$N^6$-chloroadenosine-5'-uronamide

$N^6$-chloro-2',3'-isopropylideneadenosine-5'-carboxylic acid (4.4 g, 12.9 mmol), triethylamine (1.64 mL, 11.7 mmol) isopropenyl chloroformate (1.28 mL, 11.7 mmol), and methylene chloride (50 mL) are combined under argon at -10 °C and stirred for about 2 minutes. Ethylamine (0.77 mL, 11.7 mmol) is added to the reaction mixture and stirring continued for an additional 1 minute. The reaction mixture is partitioned between methylene chloride and saturated sodium bicarbonate. The aqueous layers are washed with methylene chloride (3X). The combined organic layers are washed with brine and dried over sodium sulfate, filtered, evaporated in vacuo and the residue purified by flash chromatography on silica gel, eluting with 3% MeOH/$CHCl_3$, to give 5'-N-ethyl-2',3'-isopropylidene-$N^6$-chloroadenosine-5'-uronamide, 1 H NMR (300 MHz, (CDCl3) d 8.75 (s, 1H), 8.23 (s, 1H), 6.20 (d, 1H), 5.50 (dd, 2H), 4.73 (d, 1H), 3.01 (m, 2H), 1.63 (s, 1H), 1.41 (s, 3H), 0.77 (t, 3H).

## EXAMPLE 2

Preparation of (1S,2R,35,4R)-2,3-dihydroxy-4-[6-[1-(4-trifluormethylpyridin-2-yl)pyrrolidin-3(S)-ylamino]purin-9-yl] cyclopentanecarboxylic acid isopropylamide

Step (1)

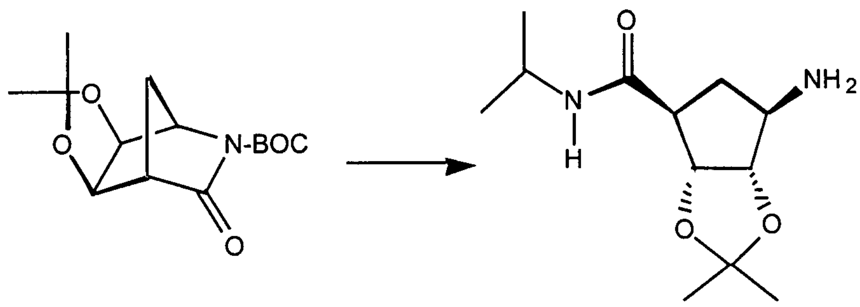

(i) (ii)

15.5 g (54.6 mmol) N-BOC-5,6-Dimethylenedioxy-2-azabicyclo[2.2.1]heptan-3-one (i) (prepared as in Step (6) of Example 3, below) is dissolved in 16 mL isopropyl amine and the mixture stirred at room temperature for about 2 hours. The mixture is evaporated in vacuo, and the residue azeotroped with chloroform to give a white solid. This solid is dissolved in 250 mL ethyl acetate, the solution cooled to 0°C, and hydrogen chloride gas is bubbled into the solution, with cooling for about 15 minutes. The solution is then stirred at room temperature for about 4 hours. The solution is evaporated in vacuo, and azeotroped with methanol, then chloroform, to give the amine product as the hydrochloride salt. The hydrochloride salt is partitioned between chloroform and sodium bicarbonate solution, and the organic layer washed with brine, dried, filtered and one equivalent of benzoic acid is added. The solvent is removed in vacuo and the residue triturated in ether to give the desired amine (ii) depicted above as the benzoate salt, m.p. 183-184°C.

Step (2) Preparation of

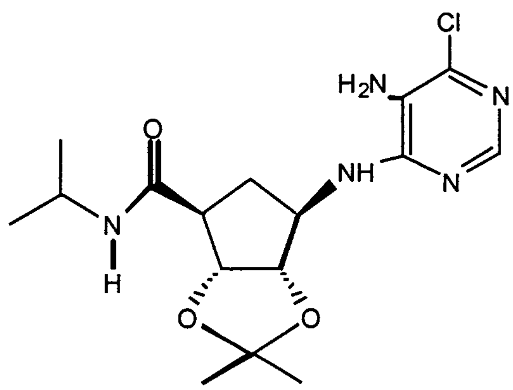

(iii)

54 mmol of the product (ii) from Example 2 Step (1) above is dissolved in 110 mL n-butanol and 9.7g 5-amino-4,6-dichloropyrimidine, then 23 mL triethylamine were added and the mixture heated at reflux for about 18 hours. The mixture is cooled, diluted with chloroform and saturated ammonium chloride solution. The aqueous layer is extracted three times chloroform, then twice with 10% isopropyl alcohol/chloroform. The organic layers are combined, dried over sodium sulfate, filtered, concentrated to an oil (iii) which is used, without further treatment for the next step.

Step (3) Preparation of

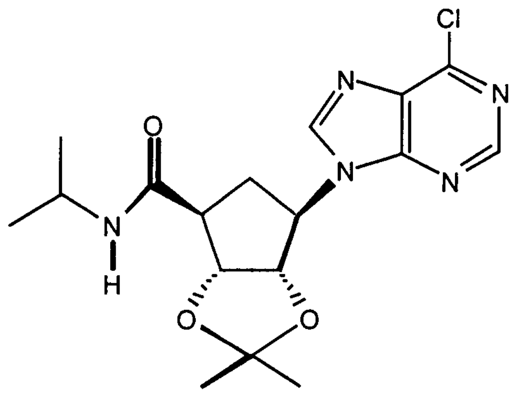

(iv)

The product (iii) from Example 2 Step (2) above is taken up in 150 mL n-butyl acetate and 11.2 g formamidine acetate is added. The mixture is heated at reflux under argon for about 9 hours, adding three 5.56 g portions of formamidine acetate at two, four, and six hours. The mixture is cooled, diluted with ethyl acetate, washed with brine, water, brine, dried over sodium sulfate, filtered, concentrated in vacuo, and the residue purified by flash chromatography, eluting with 40-80 % ethyl acetate in hexane, to give the desired chloropurine product (iv) depicted above.

Step (4) Preparation of

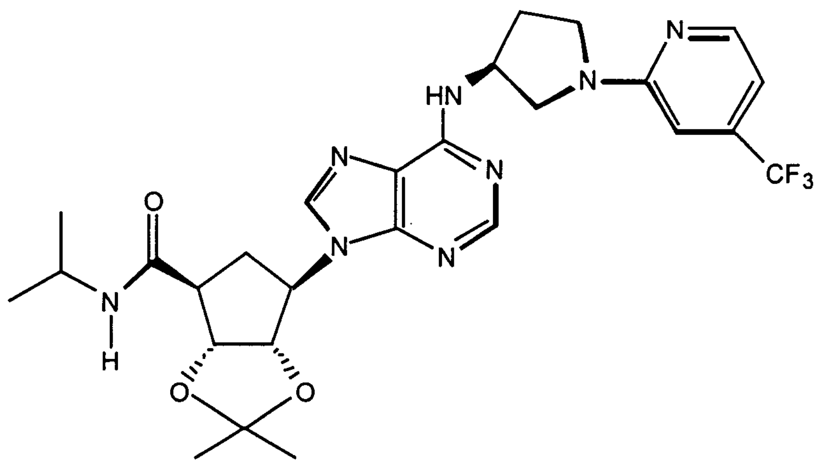

(v)

400 mg (1.05 mmol) of the product (iv) from Example 2 Step (3) above, 0.22 mL (1.57 mmol) triethylamine, and 270 mg (1.16 mmol) 2-[(3S)-3-aminopyrrolidin-1-yl]-4-trifluoromethylpyridine (prepared as in Example 3, Steps 1 to 5, below) were dissolved together in 3 mL ethanol, and the solution heated at reflux, under argon, for about 20 hours. The mixture is evaporated in vacuo and the residue partitioned between chloroform and saturated sodium bicarbonate solution. The aqueous layer is extracted with 4 portions of chloroform and the combined organic dried over sodium sulfate, filtered, evaporated in vacuo. The residue is purified by flash chromatography, applying the sample in methylene chloride/ethyl acetate (1:1), and eluting with 0 to 3% methanol in ethyl acetate, to give the above-depicted product (v).

Step (5) The product from Example 2 Step (4) above is dissolved in 2 mL methanol/tetrahydrofuran (1:1), and 3.3 mL 1.5 N aqueous hydrochloric acid is added, and the solution stirred at room temperature for about 20 hours. The mixture is evaporated in vacuo. This resulting residue is taken up in 10 mL 15% isopropyl alcohol/chloroform, 1 mL 1 N sodium hydroxide solution, and 9 mL saturated sodium bicarbonate solution. The layers are separated and the aqueous extracted with 4 x 5 mL portions of 15% isopropyl alcohol/chloroform. The combined organic layer is dried over sodium sulfate, filtered, evaporated in vacuo to give (1S,2R,3S,4R)-2,3-dihydroxy-4-[6-[1-(4-trifluormethylpyridin-2-yl)pyrrolidin-3(S)-ylamino]purin-9-yl]cyclopentanecarboxylic acid isopropylamide, m.p. 227-228°C.

EXAMPLE 3

Preparation of (1S,2R,3R,5R)-3-methoxymethyl-5-[6-[1-(5-trifluoromethylpyridin-2-yl)pyrrolidin-3(S)-ylamino]-purin-9-yl]cyclopentane-1,2-diol

Step (1) 20g (232 mmol) of (3S)-(-)-3 aminopyrrolidine and 26 mL (255 mmol, 1.1 eq) benzaldehyde are combined in 250 mL toluene and refluxed, removing water with a Dean-Stark trap, for about 4.5 hours. The mixture is cooled to 0°C and 55.7g (255.2 mmol, 1.1 eq) di-tert-butyl dicarbonate added, then stirred at room temperature. The mixture is concentrated in vacuo, stirred with $KHSO_4$ solution, extracted 3 times with ether. The aqueous layer is made alkaline and extracted with $CH_2Cl_2$. The organic layer is washed with brine and dried over $MgSO_4$, filtered, and evaporated in vacuo to give N1-BOC-(3S)-(-)-3 aminopyrrolidine.

Step (2) 34.25 g (183.9 mmol) of the product from Example 3 Step (1) above is dissolved in 200 mL $CH_2Cl_2$ and 25 mL (183.9 mmol, 1 eq) of triethylamine is added. Under a nitrogen atmosphere, 34.7 mL (367.8 mmol, 2 eq) of acetic anhydride is added dropwise, the mixture stirred at room temperature, partioned with $NaHCO_3$ solution/ $CH_2Cl_2$. The organic layer is washed with brine, dried over $MgSO_4$, filtered, evaporated in vacuo, and the product purified by flash chromatography, eluting with 2-8% methanol in methylene, to give N1-BOC-(3S)-(-)-3-acetylaminopyrrolidine.

Step (3) 39.2 g (171.7 mmol) of the product from Example 3 Step (2) above is dissolved in 400 mL $CH_2Cl_2$ and 26.46 mL (343.4 mmol 2 eq) trifluoroacetic acid (hereinafter "TFA") is dropwise at at 0°C under a nitrogen atmosphere. The mixture is heated to reflux, adding another 26 mL, then another 10 mL of TFA, refluxed for about an additional 3 hours, then evaporated under high vacuum to remove TFA. The residue was stirred with Amberlite IRA-400 basic resin (hereinafter "basic resin"), filtered, the filtrate dissolved in methanol, filtered slowly through basic resin, and the filtrate evaporated to give (3S)-(-)-3-acetylaminopyrrolidine.

Step (4) 4g (31.2 mmol) of the product from Example 3 Step (3) above and 5.19 (40.6 mmol) 2-chloro-5-trifluoromethylpyridine are combined in 50 mL ethanol and 13 mL (93.6 mmol, 3 eq) triethylamine are added. The mixture is refluxed for about 18 hours, concentrated in vacuo and the residue partitioned between methylene chloride and sodium bicarbonate solution. The organic layer is washed with brine, dried over magnesium sulfate, filtered, evaporated in vacuo, and the residue purified by flash chromatography, eluting with 2-5% methanol in methylene chloride, to give 2-[(3S)-3-acetylaminopyrrolidin-1-yl]-5-trifluoromethylpyridine, as a solid.

Step (5) 7.52 g (27.5 mmol) of the product from Example 3 Step (4) above is combined with 75 mL 6N aqueous hydrochloride acid and the mixture refluxed for about 18 hours. The mixure is cooled to room temperature, neutralized with solid sodium bicarbonate, partitioned between dilute sodium hydroxide solution and methylene chloride. The organic layer is washed with brine, dried over magnesium sulfate, filtered, evaporated in vacuo to give 2-[(3S)-3-aminopyrrolidin-1-yl]-5-trifluoromethylpyridine.

Step (6)

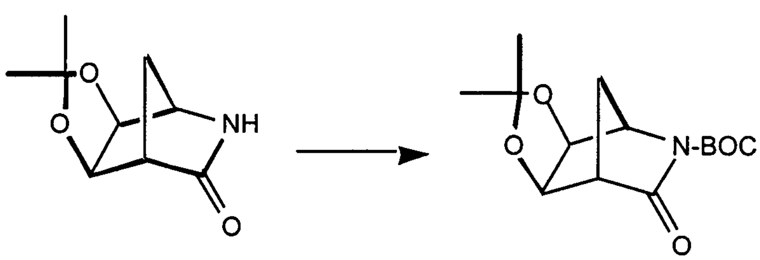

(vi) (i)

22.5 g (0.123 mol) (-)-5,6-Dimethylenedioxy-2-azabicyclo[2.2.1]heptan-3-one (vi), 1.5 g 4-dimethylaminopyridine (hereinafter "DMAP"), 12.4 g triethylamine, and 37.5 g di-tert-butyl dicarbonate are combined in methylene chloride and stirred at room temperature for about 18 hours. The mixture is washed with 1N hydrochloric acid, 5% sodium bicarbonate solution, brine, dried over sodium sulfate, filtered, concentrated in vacuo and the residue recrystallized from isopropyl alcohol to give N-BOC-5,6-Dimethylenedioxy-2-azabicyclo[2.2.1]heptan-3-one (i).

Step (7)

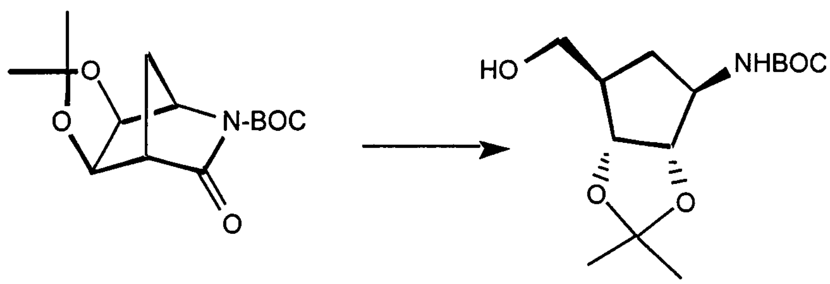

(i) (vii)

35.6g (0.125 mol) of the product (i) from Example 3 Step (6) above is combined with 400 mL methanol. With rapid stirring and cooling, under argon purge, a total of 23.8g (0.63 mol) sodium borohydride is added in three equal portions over a period of about 2 hours. The mixture is concentrated in vacuo and partitioned between 200 mL water and 300 mL ethyl acetate. The aqueous layer is extracted twice more with ethyl acetate and the combined organic solution washed with water, brine, dried over sodium sulfate, filtered, concentrated in vacuo to give N-BOC-1-amino-2,3-dimethylenedioxy-4-hydroxymethylcyclopentane (vii).

Step (8)

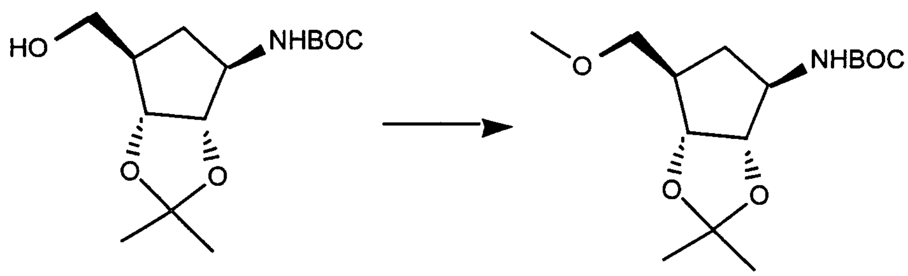

(vii) (viii)

50 g of the product (vii) from Example 3 Step (7) above is placed in 150 mL benzene. 8.8 ml methyl iodide and 33 g silver oxide are added and the mixture refluxed for about 18 hours. Another 25 g of silver oxide and another 50 mL of methyl iodide are added portionwise over about 6 hours and the mixture refluxed for about 18 hours. The mixture is filtered through Celite and the filter cake washed with ethyl acetate. The combined filtrate is concentrated in vacuo and the residue crystallized from hexane to give the desired methoxymethyl compound (viii) depicted above.

Step (9)

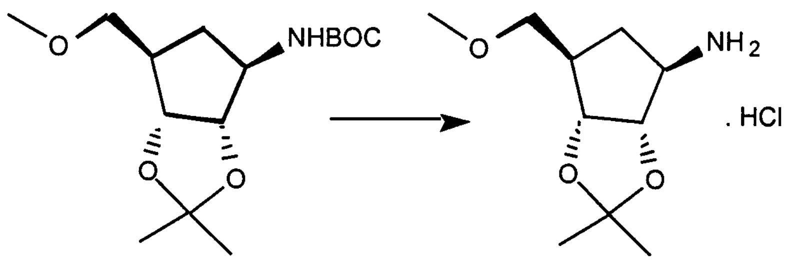

(viii) (ix)

Under argon, 31.6g of the product (viii) from Example 3 Step (8) above is dissolved in 250 mL warm anhydrous ethyl acetate. The solution is cooled in an ice bath and hydrogen chloride gas is bubbled through the solution for about 6 minutes. The mixture is allowed to warm to room temperature and stirred for about 3 hours, then concentrated in vacuo to give the desired amine hydrochloride (ix) depicted above.

Step (10)

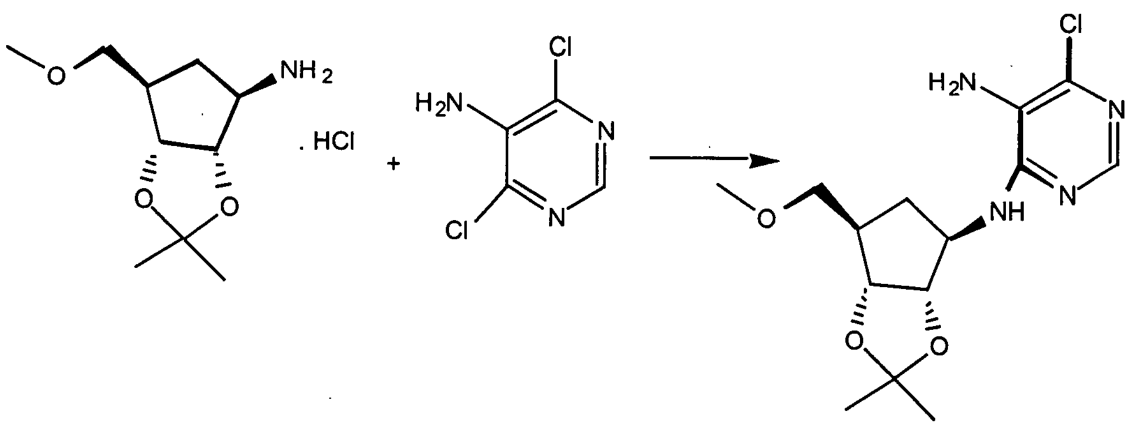

(ix) (x) (xi)

24.2 g of the product from (ix) Example 3 Step (9) above and 42.8 g sodium bicarbonate are combined in 100 mL n-butanol, under argon, and 20.1 g 5-amino-4,6-dichloro pyrimidine is added. The mixture is heated at reflux for about 20 hours, then concentrated in vacuo. The residue is partioned between ethyl acetate and water and the ethyl acetate layer washed with brine, dried over magnesium sulfate, filtered, concentrated in vacuo. The residue in 30% ethyl acetatate in hexane, passed through a large flash silica gel wash column, and the column is washed with 50% ethyl acetate/hexane and the combined filtrates concentrated in vacuo to give the desired pyrimidinylaminocyclopentane product (xi) depicted above.

Step (11)

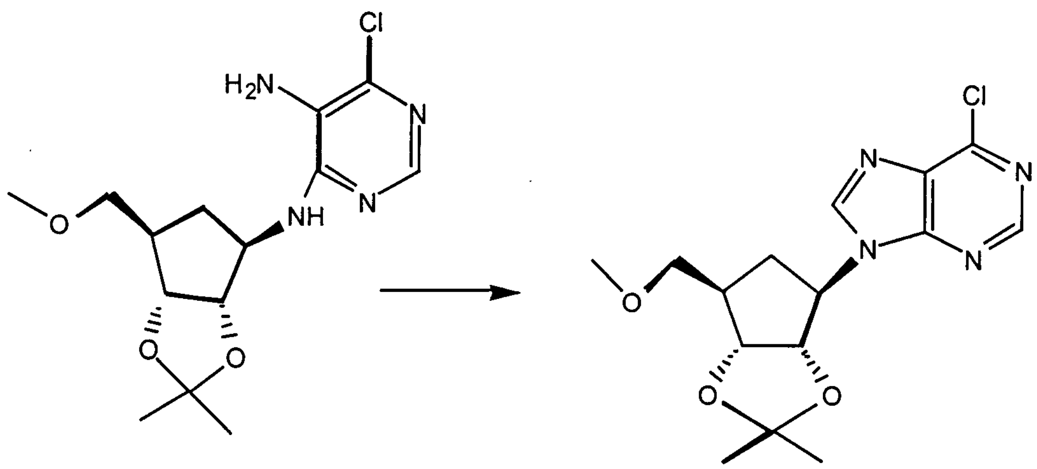

(xi) (xii)

26.7 g of the product (xi) from Example 3 Step (10) above is combined with 125 mL n-butyl acetate under argon. 33.5g formamidine acetate added and mixture heated at reflux for-about 3 hours, until thin layer chromatography shows reaction is complete. The mixture is cooled, partitioned between ethyl acetate and brine and the ethyl acetate layer dried over magnesium sulfate, filtered, concentrated in vacuo. The residue was purified by flash chromatography, eluting with 30-50% ethyl acetate in hexane, to give the chloropurine product (xii) depcited above.

Step (12) Preparation of

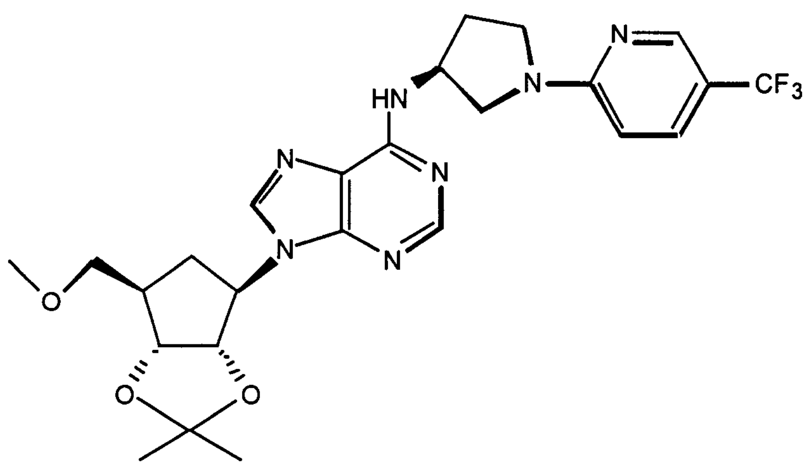

(xiii)

7.75 g (22.9 mmol) of the product (xii) from Example 3 Step (11) above and 6.35 g (27.4 mmol) 2-[(3S)-3-aminopyrrolidin-1-yl]-5-trifluoromethylpyridine are combined in 20 mL ethanol and 6.33 mL triethylamine added. The mixture is heated in a sealed vessel at 105°C for about 4 hours. The mixture is cooled, evaporated in vacuo, partitioned between methylene chloride and sodium bicarbonate solution. The organic layer is dried over magnesium sulfate, filtered, concentrated in vacuo, and the residue purified by flash chromatography, eluting with 4% methanol in methylene chloride, to give the product (xiii) indicated.

Step (13) 10.81g (20.3 mmol) of the product (xiii) from Example 3 Step (12) above is combined with 90 mL trifluoroacetate and 10 mL water, and the mixture stired at room temperature for about 30 minutes. The TFA is evaporated

off at high vacuum and the residue partitioned between methylene chloride and sodium bicarbonate solution. The methylene chloride solution is washed with sodium bicarbonate solution, brine, isopropyl alcohol is added and the solution dried over magnesium sulfate, filtered, concentrated in vacuo, and the residue flash chromatographed, eluting with 5-10% methanol in methylene chloride. The appropriate fractions are collected, concentrated, and the residue crystallized from acetonitrile to give (1S,2R,3R,5R)-3-methoxymethyl-5-[6-[1-(5-trifluoromethylpyridin-2-yl)pyrrolidin-3(S)-ylamino]-purin-9-yl]cyclopentane-1,2-diol, m.p. 166-168°C.

EXAMPLE 4

Preparation of (2R,3S,4R,5R)-2-hydroxymethyl-5-[6-[1-(5-trifluoromethylpyridin-2-yl)-pyrrolidin-3(R)-ylamino]-purin-9-yl]tetrahydrofuran-3,4-diol

267 mg 2-[(3R)-3-aminopyrrolidin-1-yl]-5-trifluoromethylpyridine, 331 mg 6-chloropurineriboside, 233 mg triethylamine, and 0.5 mL ethanol are combined and heated in a sealed vessel at 100°C for about 5 hours. The mixture is cooled, partioned between methylene chloride (with some isopropyl alcohol added) and sodium bicarbonate. The organic layer is washed with brine, dried over magnesium sulfate, evaporated, and the residue purified by flash chromatography, eluting with 5% methanol in methylene chloride, to give (2R,3S,4R,5R)-2-hydroxymethyl-5-[6-[1-(5-trifluoromethylpyridin-2-yl)-pyrrolidin-3(R)-ylamino]purin-9-yl]tetrahydrofuran-3,4-diol, as the hemihydrate, m.p. 166-170°C.

EXAMPLE 5

Preparation of (1S,2R,3R,5R)-5-(-[6-[1-(4-trifluoromethylphenyl)-pyrrolidin-3(S)-ylamino]-purin-9-yl]-3-hydroxymethylcyclopentane-1,2-diol

Step (1) 1.00 g (11.6 mmol) 3(S)-(-)-3-aminopyrrolidine, 1.35 mL (9.66 mmol) 4-bromobenzotrifluoride, 2.69 g (29 mmol) sodium tert-butoxide, and 1.01g (1.16 mmol) $PdCl_2(P[o\text{-tolyl}]_3)_2$ (prepared as in U.S. Patent No. 4,196,135, incorporated herein by reference) are combined in 30 mL toluene, and the mixture heated in a sealed vessel at 100°C for about 40 hours. The mixture was cooled, filtered, evaporated in vacuo and the residue purified by flash chromatography, eluting with 10:1 to 7:1 methylene chloride/ethanol, to give 1-(4-trifluoromethyl)phenyl-(3S)-pyrrolidin-3-ylamine.

Step (2)

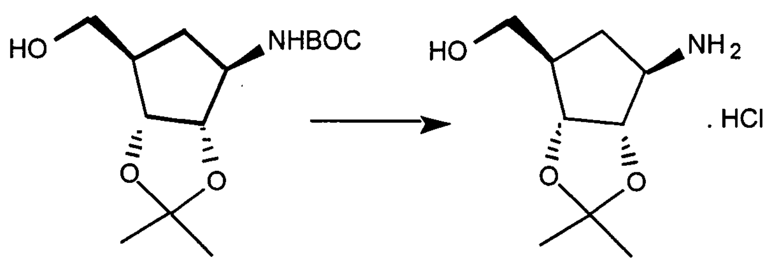

(vii) (xiv)

A solution of 24.7 mL (0.61 mol) methanol and 50 mL ethyl acetate is cooled to 0°C, under argon. 43.3 mL (0.61 mol) acetyl chloride is added portionwise and the solution allowed to come to room temperature over about 45 minutes. This solution is again cooled in ice and a solution of 50.0g N-BOC-1-amino-2,3-dimethylenedioxy-4-hydroxymethyl cyclopentane (vii) in 100 mL ethyl acetate is added over a period of about 45 minutes. The solution is allowed to come to room temperature, then evaporated in vacuo to give the desired amine hydrochloride (xiv) depicted above.

Step (3) Preparation of

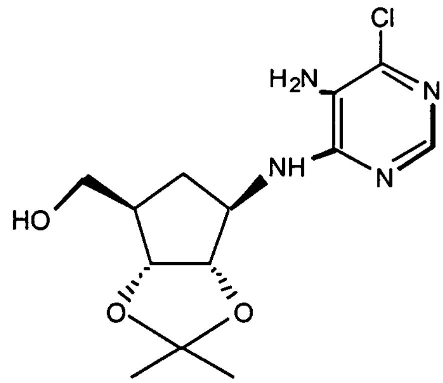

(xv)

38.9g of the product (xiv) from Example 5 Step (2) above and 73 g sodium bicarbonate are combined in 150 mL n-butanol under argon, and the mixture stirred at room temperature for about 30 minutes. 34.2 g 5-amino-4,6-dichloropyrimidine is added and the mixture stirred at reflux for about 19 hours. The mixture is concentrated in vacuo, and the residue taken up in ethyl acetate and water. The aqueous layer is extracted with ethyl acetate and the combined organic washed with brine, filtered, concentrated in vacuo. The residue is purified by flash chromatography, eluting with a gradient of 30% to 100% ethyl acetate in hexane, to give the desired substituted chloropyrimidine (xv) depicted above.

Step (4) Preparation of

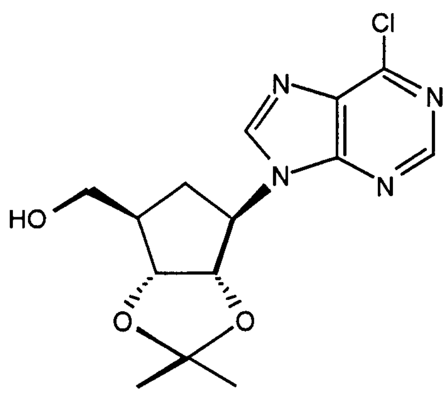

(xvi)

37.9 g of the product (xv) from Example 5 Step (3) above and 25.1 g formamidine acetate are combined in 250 mL n-butyl acetate and the mixture heated at reflux, under argon, for about 2 hours, adding an additional 12.5 g formamidine acetate after about 1 hour, and an additional 10 g after about 1.5 hours. The mixture is cooled, partitioned between ethyl acetate and brine, the brine extracted with 3 portions of ethyl acetate, and the combined organic dried over magnesium sulfate, filtered, evaporated in vacuo. The residue is purified by crystallization from ethyl acetate/hexane to give the above-depicted chloropurine (xvi). The residue from concentration of the mother liquor can be purified by flash chromatography, eluting with 80 to 100% ethyl acetate in hexane to improve recovery.

Step (5) Preparation of

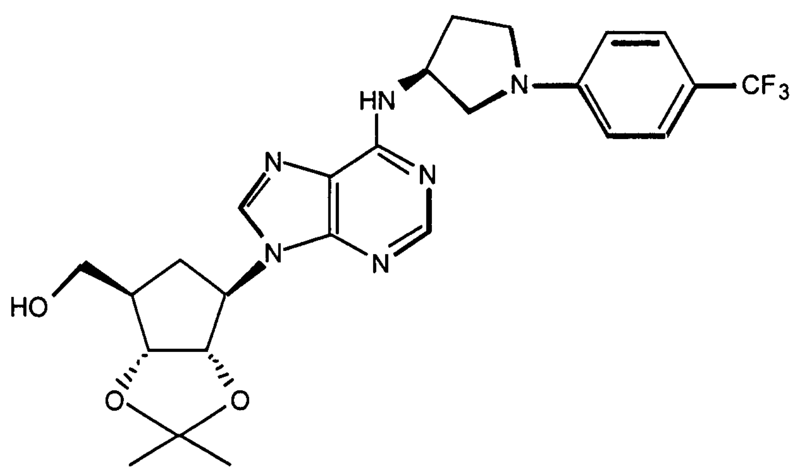

(xvii)

0.225 g (0.693 mmol) of the product (xvi) from Example 5 Step (4) above, 0.239 g (1.04 mmol) 1-(4-trifluoromethyl)phenyl-(3S)-pyrrolidin-3-ylamine, from Step (1) above, and 0.582 g (6.93 mmol) sodium bicarbonate are combined in 20 mL ethanol and heated at reflux for about 60 hours. The mixture is filtered, concentrated in vacuo, and the residue purified by flash chromatography, eluting with a gradient of methylene chloride/ethanol, 30:1 to 10:1, to give the pyrrolidinylamine (xvii) depicted above.

Step (6) 0.234 g of the product from Example 5 Step (5) above is dissolved in 10 mL trifluoroacetic acid and the solution stirred at room temperature overnight. The solution is evaporated in vacuo, and the residue purified by flash chromatography, eluting with methylene chloride/ethyl acetate (10:1) to give (1S,2R,3R,5R)-5-(-[6-[1-(4-trifluoromethylphenyl) -pyrrolidin-3(S)-ylamino]-purin-9-yl]-3-hydroxymethylcyclopentane-1,2-diol, m.p. 111-114°C.

EXAMPLE 6

Preparation of 4(S)-1-benzyl-4-[9-(2,3-dihydroxy-4-hydroxymethylcyclopentyl)-9H-purin-6-ylamino]pyrrolidin-2-one

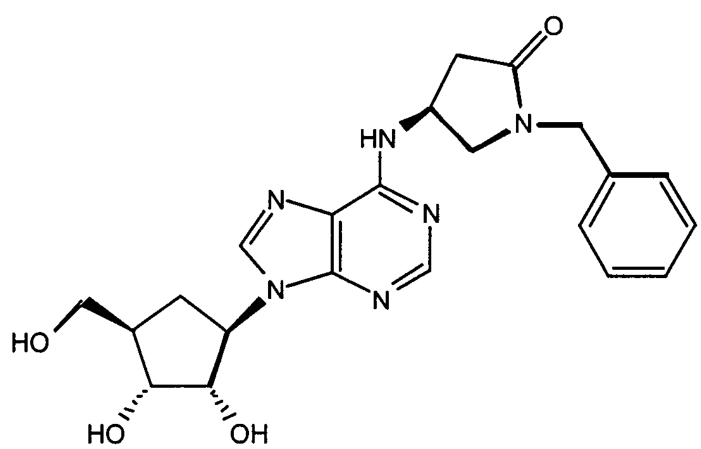

(xviii)

Step (1) Preparation of

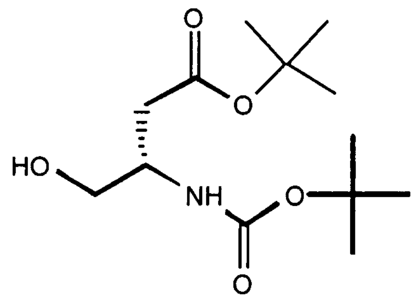

(xix)

7.1 g (24.5 mmol) N-t-BOC-L-aspartic acid β-t-butyl ester is dissolved in 120 mL tetrahydrofuran. The solution is cooled to 0°C and 2.73 g (27 mmol) triethylamine, then 2.66 g (24.5 mmol) ethyl chloroformate is added. The solution is stirred for about 30 minutes, and a solution of 3.71 g (98.2 mmol) sodium borohydride in water is added. The mixture is stirred at room temperature for about 17 hours, concentrated in vacuo and the residue diluted with ethyl acetate, and the organic layer washed with 1 N hydrochloric acid, 10% sodium carbonate, brine, then dried over magnesium sulfate, filtered, concentrated in vacuo and the residue purified by flash chromatography, eluting with 30% to 50% ethyl acetate in hexane, to give 3(S)-t-butyl-3-BOC-amino-4-hydroxy-n-butanoate (xix).

Step (2) Preparation of

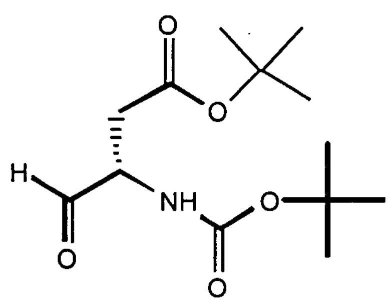

(xx)

A solution of 0.73 g of dimethylsulfoxide in 9 mL of methylene chloride is cooled to -70°C and 31 mL of a 2M solution of oxalyl chloride in methylene chloride is added dropwise. The solution is stirred for about 15 minutes and a solution of 0.85 g of 3(S)-t-butyl-3-BOC-amino-4-hydroxy-n-butanoate (xix) in 5 mL methylene chloride is added. After stirring for about 45 minutes, 1.88g triethylamine is added. The solution is allowed to warm to room temperature, stirred for about 30 minutes, then diluted with ethyl acetate. The solution is washed with 1N hydrochloric acid, 10% sodium carbonate, brine, dried over magnesium sulfate, filtered, concentrated in vacuo, to give give 3(S)-t-butyl-3-BOC-amino-4-oxo-n-butanoate (xx).

Step (3)

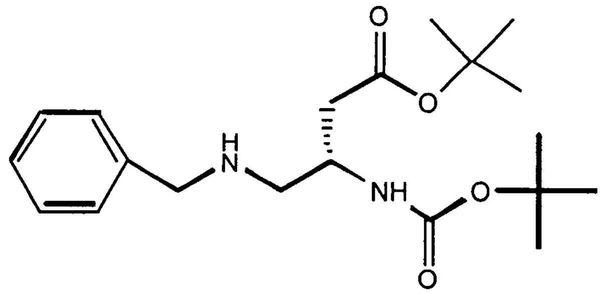

(xxi)

The product (xx) from Example 6 Step (2) above is dissolved in 9 mL methanol and 1.34g benzyl amine hydrochloride, then 0.94 g triethylamine, then 200 mg 3 Å molecular seives. The solution is stirred for about 45 minutes and a solution of 0.23 g zinc chloride and 0.22 g sodium cyanoborohydride in 5 mL methanol is added. The solution is stirred for about 4 hours, 2 mL 1N sodium hydroxide, then 10 mL water are added, the mixture concentrated to about one-half volume, and extracted with ethyl acetate. The ethyl acetate solution is washed with 10% sodium carbonate solution, brine, dried over magnesium sulfate, filtered, concentrated in vacuo, and the residue purified by flash chromatography, eluting with 30% to 40% ethyl acetate in hexane, to give the benzyl amine (xxi) depicted above.

Step (4) 0.90 g of the product from Example 6 Step (3) above is dissolved in 12 mL of toluene/acetic acid (10:1), and the solution refluxed for about 1.5 hours. The mixture is concentrated in vacuo, and the residue purified by flash chromatography, eluting with 25%-35% ethyl acetate in methylene chloride, to give 1-benzyl-4(S)-BOC-amino-2-pyrrolidinone.

Step (5) 0.64g of the product from Example 6 Step (4) above is dissolved in 20 mL ethyl acetate and the solution cooled to 0°C. Hydrogen chloride gas is bubbled into the solution for about 5 minutes, and the mixture stirred at room temperature for about 18 hours. Ether is added to the mixture and the solid collected by filtration to give 1-benzyl-4(S)-amino-2-pyrrolidinone hydrochloride.

Step (6) 0.33 g of the protected chloropurine from Example 5, Step (4) above, 0.26 g 1-benzyl-4(S)-amino-2-pyrrolidinone hydrochloride, and 0.29 g triethylamine are combined in 10 mL ethanol and the mixture heated at reflux for about 50 hours. The mixture is concentrated in vacuo and the residue dissolved in 20 mL 1 N hydrochloric acid and stirred at room temperature for about 1 hour. The mixture is concentrated in vacuo and the residue purified by preparative HPLC, eluting with a gradient of 10% acetonitrile to 60% acetonitrile in water, containing 0.1% trifluoroacetic acid. The appropriate fractions were combined, concentrated, and the residue dissolved in 20 mL 1N hydrochloric acid, the solvent evaporated in vacuo, and this repeated twice more. This residue was dissolved in methanol, the solvent evaporated in vacuo, and the residue triturated in ether to give 4(S)-1-benzyl-4-[9-(2,3-dihydroxy-4-hydroxymethylcyclopentyl)-9H-purin-6-ylamino]pyrrolidin-2-one as the hydrochloride trihydrate, m.p. 100°C (dec.)

EXAMPLE 7

Preparation of (1S,2R,3R,5R)-3-hydroxymethyl-5-[6-[1-(4-nitrophenyl)pyrrolidin-3-ylamino]-purin-9-yl]cyclopentane-1,2-diol

Step (1) 4-nitrophenol (1.0g, 7.19 mmol) and triethylamine (3 mL, 21.6 mmol) were dissolved together in anhydrous methylene chloride (10 mL), and the solution cooled to -15°C. Trifluoromethanesulfonic anhydride (1.81 mL, 10.8 mmol) is added and the mixture stirred at -15°C for about 30 minutes. The mixture is diluted with methylene chloride, washed with sodium bicarbonate solution and brine, the organic layer dried over magnesium sulfate, filtered, and concentrated in vacuo. The residue is purified by flash chromatography, eluting with methylene chloride, to give

4-nitrophenyl trifluoromethanesulfonate as a light yellow solid.

Step (2) 3(S)-amino-1-benzylpyrrolidine (3.0g, 17.0 mmol) and triethylamine (2.50 mL, 17.9 mmol) are dissolved together in anhydrous methanol (17 mL), under nitrogen, and ethyl trifluoroacetate (2.53 mL, 21.3 mmol) is added dropwise. The solution is stirred for about 18 hours, evaporated in vacuo, and the residue taken up in methylene chloride. The solution is washed with sodium bicarbonate solution, brine, dried over magnesium sulfate, filtered, concentrated in vacuo to give 1-benzyl-3(S)-trifluoroacetylaminopyrrolidine.

Step (3) Under nitrogen, 1-benzyl-3(S)-trifluoroacetylaminopyrrolidine (4.59g, 16.7 mmol) is dissolved in anhydrous methanol (50 mL) and di-tert-butyl dicarbonate (3.68g, 16.7 mmol) and 10% palladium on carbon (0.90g) are added. The mixture is then stirred under hydrogen under atmospheric pressure for about 5 hours. The mixture is filtered through Celite®, rinsing with methanol, and the filtrate evaporated in vacuo, The residue was purified by flash chromatography, eluting with 5% methanol in methylene chloride to give 1-BOC-3(S)-trifluoroacetylaminopyrrolidine.

Step (4) 1-BOC-3(S)-trifluoroacetylaminopyrrolidine (4g) is dissolved in methylene chloirde (130 mL) and trifluroacetic acid (19 mL) is added. The solution is stirred at room temperature for about 1 hour, then concentrated in vacuo. The residue is partitioned between methylene chloride and saturated sodium bicarbonate solution. The layers are separated and the aqueous extracted with ethyl acetate. The combined organic is dried over magnesium sulfate, filtered, evaporated in vacuo to give 3(S)-trifluoroacetylaminopyrrolidine.

Step (5) 4-Nitrophenyl trifluoromethanesulfonate (0.423g, 1.56 mmol) and triethylamine (0.217 mL, 1.56 mmol) are dissolved together in anhydrous acetonitrile (15 mL) and 3(S)-trifluoroacetylaminopyrrolidine (0.852g, 4.68 mmol) is added and the mixture heated at reflux for about 18 hours. The mixture is cooled, concentrated in vacuo and the residue purified by flash chromatography, eluting with a gradient of 25% to 50% ethyl acetate in hexane to give 1-(4-nitro)phenyl-3(S)-trifluoroacetylaminopyrrolidine.

Step (6) 1-(4-Nitro)phenyl-3(S)-trifluoroacetylaminopyrrolidine (0.334g, 1.10 mmol) is combined with a saturated solution of potassium carbonate in methanol/water (2:3) (20 mL), and the mixture heated at 55°C for about two hours, then at room temperature for about 18 hours. The mixture is concentrated in vacuo and the residue taken up in water (10 mL). The aqueous is extracted with ethyl acetate, and the organic dried over magnesium sulfate, filtered, evaporated in vacuo to give 3(S)-amino-1-(4-nitro)phenylpyrrolidine.

Step (7) Using essentially the procedures of Example 3, Steps 12 and 13, and Example 5, Steps 5 and 6, (1S,2R,3R,5R)-3-hydroxymethyl-5-[6-[1-(4-nitrophenyl)pyrrolidin-3-ylamino]-purin-9-yl]cyclopentane-1,2-diol, m.p. 119-120°C, is prepared from 3(S)-amino-1-(4-nitro)phenylpyrrolidine.

Using essentially the procedures of the Reaction Schemes and Examples as described hereinabove, the following compounds for use of the invention are prepared from the appropriate starting materials:

(2R,3R,4S,5R)-2-hydroxymethyl-5-[6-[1-(5-chloropyridin-2-yl)-pyrrolidin-3(S)-ylamino]-purin-9-yl]-tetrahydrofuran-3,4-diol, m.p. 154-156°C;

(2R,3R,4S,5R)-2-hydroxymethyl-5-[6-[1-(5-trifluoromethylpyridin-2-yl)-pyrrolidin-3(S)-ylamino]-purin-9-yl]tetrahydrofuran-3,4-diol, m.p. 153-156°C;

(2R,3R,4S,5R)-2-hydroxymethyl-5-[6-[1-(4-trifluoromethylpyridin-2-yl)-pyrrolidin-3(S)-ylamino]-purin-9-yl]tetrahydrofuran-3,4-diol, m.p. 187-190°C;

(2R,3R,4S,5R) 2-hydroxymethyl-5-[6-[1-(5-bromopyridin-2-yl)-pyrrolidin-3(S)-ylamino]-purin-9-yl]-tetrahydrofuran-3,4-diol, 153-154°C;

(2R,3R,4S,5R)-2-hydroxymethyl-5-(6-(1-(4-nitrophenyl)-pyrrolidin-3(S)-ylamino)-purin-9-yl) tetrahydrofuran-3,4-diol, m.p. 230-232°C;

(2R,3R,4S,5R)-2-hydroxymethyl-5-[6-(5'-trifluoromethyl-3,4,5,6-tetrahydro-2H-[1,2']-bipyridinyl-3-yl)-purin-9-yl] tetrahydrofuran-3,4-diol, m.p. 113-116°C;

(2R,3R,4S,5R)-2-hydroxymethyl-5-[6-(phenylpyrrolidin-3(S)-ylamino)-purin-9-yl]tetrahydrofuran-3,4-diol;

(2R,3R,4S,5R)-2-hydroxymethyl-5-[6-(1-pyridin-2-ylpyrrolidin-3(S)-ylamino]-purin-9-yl]tetrahydrofuran-3,4-diol, m.p. 193-195°C;

(2R,3R,4S,5R)-2-hydroxymethyl-5-[6-[1-(4-chlorophenyl)-pyrrolidin-3(S)-ylamino]-purin-9-yl]-tetrahydrofuran-3,4-diol, m.p. 121-124°C;

(2R,3R,4S,5R)-2-hydroxymethyl-5-[6-[1-(5-methylpyridin-2-yl)-pyrrolidin-3(S)-ylamino]-purin-9-yl]tetrahydrofuran-3,4-diol, m.p. 164-166°C;

(2R,3R,4S,5R)-2-hydroxymethyl-5-[6-[1-(5-thiophen-2-ylpyridin-2-yl)-pyrrolidin-3(S)-ylamino]-purin-9-yl]tetrahydrofuran-3,4-diol, 190-192°C;

(2R,3R,45,5R)-2-hydroxymethyl-5-[6-[1-(5-methylmercaptopyridin-2-yl)-pyrrolidin-3(S)-ylamino]-purin-9-yl]tetrahydrofuran-3,4-diol, m.p. 231-233°C;

(2R,3R,4S,5R)-2-hydroxymethyl-5-[6-[1-(6-methoxypyrimidin-4-yl)pyrrolidin-3(S)-ylamino]-purin-9-yl]tetrahydrofuran-3,4-diol, m.p. 251-253°C;

(2R,3R,4S,5R)-2-hydroxymethyl-5-[6-[1-(6-chloropyrimidin-4-yl)pyrrolidin-3(S)-ylamino]-purin-9-yl]-tetrahydrofuran-3,4-diol, 154-156°C;

(2R,3R,4S,5R)-2-hydroxymethyl-5-[6-[1-(6-chloropyridazin-3-yl)pyrrolidin-3-ylamino]-purin-9-yl]-tetrahydrofuran-3,4-diol, m.p. 130°C (dec.);

(2R,3R,4S,5R)-2-methoxymethyl-5-[6-[1-(5-trifluoromethylpyridin-2-yl)-pyrrolidin-3(S)-ylamino]-purin-9-yl]tetrahydrofuran-3,4-diol, m.p. 198-200°C;

(1S,2R,3S,4R)-2,3-dihydroxy-4-[6-[1-(5-trifluormethylpyridin-2-yl)pyrrolidin-3-ylamino]-purin-9-yl]cyclopentanecarboxylic acid ethylamide, m.p. 135-138°C;

(1S,2R,3R,5R)-3-hydroxymethyl-5-[6-[1-(4-nitrophenyl)piperidin-4-yl]-purin-9-yl]cyclopentane-1,2-diol, m.p. 126-128°C;

(1S,2R,3R,5R)-3-hydroxymethyl-5-[6-((3S)-pyrrolidin-3-ylamino)-purin-9-yl] cyclopentane-1,2-diol dihydrochloride, m.p. 160°C (dec);

(1S,2R,3R,5R)-3-hydroxymethyl-5-[6-[1-(5-trifluoromethylpyridin-2-yl)pyrrolidin-3(R)-ylamino]-purin-9-yl]cyclopentane-1,2-diol, 175-177°C;

(1S,2R,3R,5R)-3-hydroxymethyl-5-[6-((3R)-pyrrolidin-3-ylamino)-purin-9-yl] cyclopentane-1,2-diol, m.p. 166°C (dec);

(1R,25,3R,5R)-3-hydroxymethyl-5-[6-[1-(5-trifluoromethylpyridin-2-yl)pyrrolidin-3(S)-ylamino]-purin-9-yl]cyclopentane-1,2-diol, m.p. 110-111°C;

4(R)-1-benzyl-4-[9-(2,3-dihydroxy-4-hydroxymethylcyclopentyl)-9H-purin-6-ylamino] pyrrolidin-2-one hydrochloride, m.p. 110°C (dec);

(1R,2S,3R,5S)-5-methyl-3-[6-[1-(5-trifluoromethylpyridin-2-yl)pyrrolidin-3(S)-ylamino]-purin-9-yl]cyclopentane-1,2-diol, m.p. 114-116°C;

(1S,2R,3R,5R)-5-[6-[1-(5-bromopyridin-2-yl)pyrrolidin-3(S)-ylamino]-purin-9-yl]-3-hydroxymethylcyclopentane-1,2-diol, m.p. 169-171°C;

(1S,2R,3R,5R)-5-[6-[1-(5-chloropyridin-2-yl)pyrrolidin-3(S)-ylamino]-purin-9-yl]-3-hydroxymethylcyclopentane-1,2-diol, m.p. 118-121°C;

(1S,2R,3R,5R)-3-hydroxymethyl-5-[6-[1-(4-trifluoromethylpyridin-2-yl)pyrrolidin-3(S)-ylamino]-purin-9-yl]cyclopentane-1,2-diol, m.p. 135-137°C;

(1S,2R,3R,5R)-3-hydroxymethyl-5-[6-[1-(pyridin-2-yl)pyrrolidin-3(S)-ylamino]-purin-9-yl]cyclopentane-1,2-diol, m.p. 110-112°C;

(1S,2R,3R,5R)-3-hydroxymethyl-5-[6-[1-(quinolin-3-yl)pyrrolidin-3(S)-ylamino]-purin-9-yl]cyclopentane-1,2-diol, m.p. 135-138°C;

(1S,2R,3R,5R)-3-hydroxymethyl-5-[6-[1-S-(4-nitrophenyl)-pyrrolidin-3(S)-ylamino]-purin-9-yl]cyclopentane-1,2-diol;

(1S,2R,3R,5R)-5-[6-[1-(4,5-bistrifluorpyridin-2-yl)pyrrolidin-3(S)-ylamino]-purin-9-yl]-3-hydroxymethylcyclopentane-1,2-diol, m.p. 123-126°C;

(1S,2R,3R,5R)-3-hydroxymethyl-5-[6-[1-(phenyl)-pyrrolidin-3(S)-ylamino]-purin-9-yl]cyclopentane-1,2-diol, m.p. 97-99°C;

4-[3(S)-[9-(2,3-dihydroxy-4-hydroxymethylcyclopentyl)-9H-purin-6-ylamino]pyrrolidin-1-yl]benzonitrile, m.p. 140°C;

(1S,2R,3R,5R)-3-hydroxymethyl-5-[6-[1-(isoquinolin-1-yl)pyrrolidin-3(S)-ylamino]-purin-9-yl]cyclopentane-1,2-diol, m.p. 119-122°C;

(1S,2R,3R,5R)-5-[6-[1-(6-bromoquinolin-2-yl)pyrrolidin-3(S)-ylamino]-purin-9-yl]-3-hydroxymethylcyclopentane-1,2-diol;

(1S,2R,3R,5R)-5-[6-[1-(4-chlorophenyl)pyrrolidin-3(S)-ylamino]-purin-9-yl]-3-hydroxymethylcyclopentane-1,2-diol;

(1S,2R,3R,5R)-3-[6-[1-(3-chloro-5-trifluoromethylpyridin-2-yl)pyrrolidin-3(S)-ylamino]-purin-9-yl]-3-methoxymethylcyclopentane-1,2-diol, m.p. 140-143°C;

(1S,2R,3R,5R)-5-[6-[1-(6-chloropyrimidin-4-yl)pyrrolidin-3(S)-ylamino]-purin-9-yl]-3-hydroxymethylcyclopentane-1,2-diol, m.p. 180-182°C;

(1S,2R,3R,5R)-5-[6-[1-(6-chloropyrimidin-4-yl)pyrrolidin-3(S)-ylamino]-purin-9-yl]-3-methoxymethylcyclopentane-1,2-diol;

(1S,2R,3R,5R)-5-[6-[1-(6-chloropyridazin-3-yl)pyrrolidin-3(S)-ylamino]-purin-9-yl]-3-hydroxymethylcyclopentane-1,2-diol;

(1S,2R,3R,5R)-3-methoxymethyl-5-[6-[1-(6-methoxypyrimidin-4-yl)pyrrolidin-3(S)-ylamino]-purin-9-yl]cyclopentane-1,2-diol, m.p. 118-120°C;

(1S,2R,3R,5R)-3-isopropoxymethyl-5-[6-[1-(5-trifluoromethylpyridin2-yl)pyrrolidin-3(S)-ylamino]-purin-9-yl]cyclopentane-1,2-diol, m.p. 157-158°C;

(1S,2R,3R,5R)-3-isopropoxymethyl-5-[6-[1-(4-trifluoromethylpyridin2-yl)pyrrolidin-3(S)-ylamino]-purin-9-yl]cyclopentane-1,2-diol, 160-161°C;

(1S,2R,3R,5R)-5-[6-[1-(6-chloropyridazin-3-yl)pyrrolidin-3(S)-ylamino]-purin-9-yl]-3-methoxymethylcyclopentane-1,2-diol, m.p. 122-124°C;

(1S,2R,3R,5R)-5-[6-[1-(5-bromopyridin-2-yl)pyrrolidin-3(S)-ylamino]-purin-9-yl]-3-methoxymethylcyclopentane-1,2-diol, m.p. 110-111°C;

(1 S,2R,3R,5R)-5-[6-[1-(5-chlorpyridin-2-yl)pyrrolidin-3(S)-ylamino]-purin-9-yl]-3-methoxymethylcyclopentane-1,2-diol, m.p. 110-112°C;

(1S,2R,3R,5R)-3-methoxymethyl-5-[6-[1-(4-trifluoromethylphenyl)pyrrolidin-3(S)-ylamino]-purin-9-yl]cyclopentane-1,2-diol, m.p. 128°C;

(1S,2R,3R,5R)-5-(-[6-[1-(4-chlorophenyl)-pyrrolidin-3(S)-ylamino]-purin-9-yl]-3-methoxymethylcyclopentane-1,2-diol, m.p. 122-125°C;

(1 S,2R,3R,5R)-5-[6-[1-(3-chlorophenyl)-pyrrolidin-3(S)-ylamino]-purin-9-yl]-3-methoxymethylcyclopentane-1,2-diol, m.p. 127-130°C;

(1S,2R,3R,5R)-5-[6-[1-(3-chlorophenyl)-pyrrolidin-3(S)-ylamino]-purin-9-yl]-3-hydroxymethylcyclopentane-1,2-diol, m.p. 131-133°C;

(1S,2R,3R,5R)-3-methoxymethyl-5-[6-[1-phenylpyrrolidin-3-(S)-ylamino]-purin-9-yl]cyclopentan-1,2-diol, m.p. 106°C;

(1S,2R,3R,5R)-3-[6-(1-benzyl-pyrrolidin-3(S)-ylamino)purin-9-yl]5-hydroxymethylcyclopentane-1,2-diol, m.p. 100-102°C;

(1S,2R,3R,5R)-3-[6-(1-benzyl-pyrrolidin-3(S)-ylamino)purin-9-yl]5-methoxymethylcyclopentane-1,2-diol, m.p. 95-96°C;

(1S,2R,3S,4R)-2,3-dihydroxy-4-{6-[1-(5-trifluoromethyl-pyridin-2-yl)-pyrrolidin-3(S)-ylamino]-purin-9-yl}-cyclopentanecarboxylic acid-1(S)-methylpropylamide, m.p. 215°C (dec.); and

(1S,2R,3S,4R)-2,3-dihydroxy-4-{6-[1-(5-trifluoromethyl-pyridin-2-yl)-pyrrolidin-3(S)-ylamino]-purin-9-yl}-cyclopentanecarboxylic acid-1(R)-methylpropylamide, m.p. 206-212°C (dec.).

Aspects of the pathophysiology of insulin resistance and type 2 diabetes

Insulin resistance, which is defined as a state of reduced responsiveness to normal circulating concentrations of insulin, is a characteristic trait of type 2 diabetes and contributes to abnormalities in muscle, fat tissue and liver. Insulin resistance precedes the onset of type 2 diabetes, which develops when additional defects exist at the level of the pancreatic beta-cell. As long as the peripheral insulin resistance can be compensated by increased insulin production glucose homeostasis is balanced. Even in the absence of type 2 diabetes, insulin resistance is a key feature of other human disease states. Impaired insulin action coupled with hyperinsulinemia leads to a variety of abnormalities, including elevated triglycerides, low levels of HDL, enhanced secretion of VLDL, disorders of coagulation, increased vascular resistance, changes in steroid hormone levels, attenuation of peripheral blood flow and weight gain. Thus, insulin resistance is often associated with central obesity, hypertension, polycystic ovarian syndrome, dyslipidemia, and atherosclerosis (JCI 106, 163-164, 2000).

Insulin resistance is characterized

- in the adipose tissue by increased lipolysis, which results in increased levels of free fatty acids, which by itself contributes
  - to reduced glucose utilisation in the muscle,
  - to increased flux of fatty acids to the liver, with subsequent increased VLDL production, and
  - to impairment of insulin secretion from the beta-cell
- in the liver by increased hepatic glucose production, and VLDL secretion, which result in hyperglycemia and hypertriglyceridemia, respectively.
- in the muscle by reduced glucose utilisation rates, which contributes to hyperglycemia, Inhibition of periphral lipolysis by TGL 749 (adenosine A1 receptor agonist) results in reduction of plasma free fatty acids (primary pharmacological effect). Reduced availability of free fatty acids to the muscle increased glucose utilisation and reduced VLDL production in the liver which is paralleld by reduced plasma triglycerides (secondary pharmacological effect).

Measurement of plasma lipdis (free fatty acids, triglycerides, cholesterol) in anaesthetized rats.

Plasma lipid levels were assayed in anaesthetized rats. Briefly, rats were anaesthetized with an intraperitoneal injection of pentobarbital sodium (60 mg/kg), tracheotomized, and one jugular vein per rat was cannulated for intravenous administration (bolus injection or infusion). Anesthesia was maintained for up to 7 hours by subcutaneous infusion

of pentobarbital sodium (adjusted to the anesthetic depth of the individual animal; about 24 mg/kg/h). Body temperature was monitored with a rectal probe thermometer, and temperature was maintained at 37° C by means of a heated surgical plate. The rats were allowed to stabilize their blood levels after surgery for up to 2 hours, after which the test compound was injected intraperitoneally. Blood samples for glucose analysis (10 µl) were obtained from the tip of the tail every 15 minutes. For analysis of plasma lipids blood samples (0.3 ml) were obtained from the jugular vein either every 10 to 15 minutes for up to 2 hours, or every hour for up to 5 hours after compound administration. Standard enzymatic procedures were used to determine blood glucose (Bergmeyer, 1974).

Table 1

| Effect of Compund of Example 3 on plasma free fatty acids in anaesthetized starved male Wistar rats (Animal Study: 00-009 | | | | | | |
|---|---|---|---|---|---|---|
| | **Control** | | **Example 3 (10 mg/kg i.p.)** | | **Example 3 (30 mg/kg i.p.)** | |
| **minutes** | **Mean** | **SEM** | **mean** | **SEM** | **mean** | **SEM** |
| | | | | | | |
| **-60** | 0,57 | 0,04 | 0,61 | 0,03 | 0,52 | 0,02 |
| **10** | 0,55 | 0,04 | 0,42 | 0,03 | 0,24 | 0,02 |
| **20** | 0,59 | 0,02 | 0,26 | 0,07 | 0,14 | 0,01 |
| **30** | 0,52 | 0,04 | 0,19 | 0,05 | 0,13 | 0,01 |
| **40** | 0,50 | 0,04 | 0,19 | 0,06 | 0,10 | 0,01 |
| **60** | 0,48 | 0,07 | 0,13 | 0,05 | 0,09 | 0,01 |
| | | | | | | |
| Values are mean +/- SEM, n= 4 rats | | | | | | |

Measurement of insulin sensitivity in conscious rats.

Insulin resistant Zucker Fatty rats or Zucker Diabetic Fatty (ZDF) rats were treated for up to 3 weeks with the test compound orally once daily. Plasma parameters were obtained by retroorbital bleeding during inhalation anaesthesia on respective study days. At the end of the study rats were starved overnight and they received an insulin bolus injection (3 U/kg s.c.) and blood glucose reduction was monitored up to 6 hours. In the case of improvement of insulin sensitivity by the test compound the blood glucose reduction was much more pronounced and prolonged compared to that of the control group.

Table 2

| Insulin tolerance test (3 U/kg s.c.) in insulin resistant Zucker Fatty Rats after pretreatment with Compound of Example 3 for 3 weeks with 1 and 10 mg/kg po (Animal Study: 00-027) | | | | | | | | | |
|---|---|---|---|---|---|---|---|---|---|
| **minutes after insulin** | | **0** | **60** | **120** | **180** | **240** | **300** | **360** | **420** |
| | | | | | | | | | |
| **control** | *mean* | 5,28 | 2,38 | 2,92 | 5,87 | 6,89 | 6,20 | 5,24 | 4,93 |
| | *SEM* | 0,14 | 0,10 | 0,21 | 0,35 | 0,46 | 0,33 | 0,26 | 0,16 |
| | | | | | | | | | |
| **Example 3 (1 mg/ kg p.o.)** | | 4,60 | 1,96 | 1,59 | 1,72 | 3,02 | 5,13 | 4,98 | 4,04 |
| | | 0,16 | 0,07 | 0,09 | 0,12 | 0,25 | 0,47 | 0,26 | 0,24 |
| | | | | | | | | | |

Table 2 (continued)

| Insulin tolerance test (3 U/kg s.c.) in insulin resistant Zucker Fatty Rats after pretreatment with Compound of Example 3 for 3 weeks with 1 and 10 mg/kg po (Animal Study: 00-027) | | | | | | | | |
|---|---|---|---|---|---|---|---|---|
| **minutes after insulin** | **0** | **60** | **120** | **180** | **240** | **300** | **360** | **420** |
| **Example 3 (10 mg/ kg p.o.)** | 5,17 | 1,99 | 1,57 | 1,44 | 2,54 | 4,35 | 5,63 | 6,09 |
| | 0,22 | 0,17 | 0,16 | 0,09 | 0,27 | 0,42 | 0,47 | 0,31 |
| | | | | | | | | |
| Values are mean +/- SEM, n = 9 | | | | | | | | |

Compounds within the scope of this invention exhibit activity in standard $A_1/A_2$ receptor binding assays for the determination of adenosine receptor agonist activity in mammals. Exemplary test procedures which are useful in determining the receptor binding affinity of compounds of the present invention are described below.

A. IN VITRO ADENOSINE RECEPTOR BINDING AFFINITY DETERMINATION

$A_1$ Receptor Binding Affinity was determined by competition assay based on ligand displacement of $^3$H-CHA (cyclohexyl adenosine) [Research Biochemicals Inc., Natick, Mass.] from receptor using a membrane preparation of whole rat brain, according to the procedure of R. F. Bruns et al., Mol. Pharmacol., 29:331 (1986). Non-specific binding was assessed in the presence of 1 mM theophylline.

$A_2$ receptor binding affinity was determined by a similar assay technique, based on ligand displacement of $^3$H-CGS 21680, a known $A_2$ receptor-specific adenosine agonist, from receptor, using membranes from rat brain striatum. Non-specific binding was assessed in the presence of 20 μM 2-chloroadenosine.

The assays were run in glass test tubes in duplicate at 25∝C. Once the membranes were added, the tubes were vortexed and incubated at 25∝C for 60 minutes ($A_1$ assay) or 90 minutes ($A_2$ assay) on a rotary shaker. The assay tubes were vortexed halfway through the incubation and again near the end. The assays were terminated by rapid filtration through 2.4 cm GF/B filters using a Brandel Cell Harvestor. The test tubes were washed three times with cold 50 mM tris-HCl (pH 7.7 or 7.4), with filtration being completed within 15 seconds. The damp filter circles were placed in glass scintillation vials filled with 10 mL of Aquasol II (New England Nuclear). The vials were allowed to shake overnight on a rotary shaker and were placed into a liquid scintillation analyzer for two minute counts. $IC_{50}$ values for receptor binding, i.e. the concentration at which a compound of the invention displaced the radiolabeled standard, were obtained using a curve-fitting computer program (RS/1, Bolt, Beranek and Newman, Boston, MA).

$A_1$ Receptor Binding Affinity was determined also using a preparation of rat epididymal fat pad membranes.

Membrane Preparation: Rat epididymal fat pads are homogenized in buffer containing 0.25 M Sucrose, 10 mM Tris, 2 mM EDTA, 0.1 M phenylmethylsulfonylfluoride, and 1 μg/mL Leupeptin (200 mg wet tissue weight/mL buffer). This homogenate is placed into 50 mL centrifuge tubes and centifuged at 1000 g (3000 RPM) for 1 minute, the intermediate supematent is removed and centrifuged at 38,000 g for 15 minutes. The pellets are resuspended pellets in assay buffer (50 mM Tris and 1 mM EDTA) (300 mg original tissue weight/mL assay buffer), and 2 μl/ ml of a solution of adenosine deaminase (10 mg/ml) is added to the suspension and the suspension incubated for 30 minutes at 37°C. The suspension is centrifuged at 38,000 g for 10 minutes, the pellet washed once with 20 ml assay buffer, the resuspended in assay buffer (1.2 g original wet tissue weight / mL buffer).

Assay and Counting: Tubes are prepared as follows: Totals (total counts bound) tubes, 100 μL membrane suspension (prepared as described above), 50 μL $^3$H-cyclohexyladenosine solution (prepared by diluting a solution of approximately 1 mCi/mL, with a specific activity of approximately 29.9 Ci/mmol, with assay buffer to 100nM, hereinafter "CHA solution"), 350 μL assay buffer; Non-specific binding tubes, 100 μL membrane suspension, 50 μL CHA solution, 50 μL 100 μM 2-chloroadenosine in assay buffer, 300 μL assay buffer; Sample tubes, 100 μL membrane suspension, 50 μL CHA solution, 50 μL of a solution of the compound to be tested (which may be prepared from serial dilution in assay buffer of a DMSO solution), 300μL assay buffer; Blank tubes, 50 μL CHA solution, 450 μL assay buffer. Each tube is vortexed for 10 seconds, incubated at 23°C for two hours, and filtered using a Brandel Filtration Unit, using Whatman GF/B Filter Paper, washing twice with 5 mL 50mM Tris. The filter discs are placed in 7 mL scintillation vials, which are then filled with approximately 5 mL Ready Safe Scintillation Cocktail, and counted.

## Claims

**1.** Use of a compound of the formula

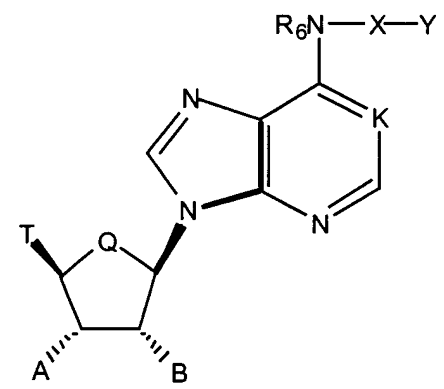

wherein:

K is N, N→O, or CH;
Q is $CH_2$ or O;
$R_6$ is hydrogen, alkyl, allyl, 2-methylallyl, 2-butenyl, or cycloalkyl;
X is

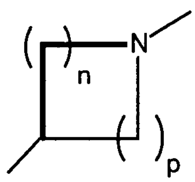

or

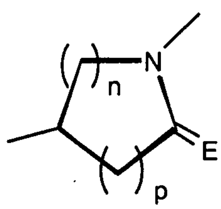

where the nitrogen of the ring of X is substituted by Y;
E is O or S;
Y is hydrogen, alkyl, aralkyl, substituted aralkyl, aryl, substituted aryl, heterocyclyl, substituted heterocyclyl, heterocyclylalkyl, or substituted heterocyclylalkyl;
n and p are independently 0, 1, 2, or 3, provided that n + p is at least 1;
T is hydrogen, alkyl, acyl, thioacyl, halo, carboxyl,

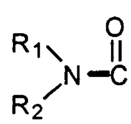

,

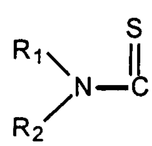

or $R_3O\text{-}CH_2$;
$R_1$, $R_2$, and $R_3$ are independently H, alkyl, or cycloalkyl;
A is hydrogen, alkyl, hydroxyalkyl, alkoxyalkyl, or OR';
B is hydrogen, alkyl, hydroxyalkyl, alkoxyalkyl, or OR";
R' and R" are independently hydrogen, alkyl, aralkyl, carbamoyl, alkyl carbamoyl, dialkylcarbamoyl, acyl, alkoxycarbonyl, aralkoxycarbonyl, aryloxycarbonyl, or, when A and B are OR' and OR", respectively, R' and R" together may form

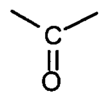, 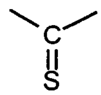, 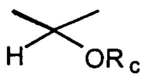

where $R_c$ is hydrogen or alkyl,

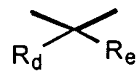

where $R_d$ and $R_e$ are independently hydrogen, alkyl, or together with the carbon atom to which they are attached may form a 1,1-cycloalkyl group;

or a pharmaceutically acceptable salt thereof, a pharmaceutically acceptable prodrug thereof, an N-oxide thereof, a hydrate thereof or a solvate thereof for producing a medicine for the treatment of the insulin resistance syndrome and diabetes.

**2.** The use of a compound according to claim 1 wherein K is N;

T is hydroxymethyl or methoxymethyl;
A and B are hydroxy;
X is

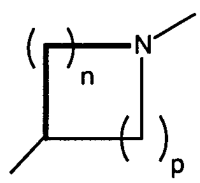;

and n + p is 3 or 4;

or a pharmaceutically acceptable salt thereof, a pharmaceutically acceptable prodrug thereof, an N-oxide thereof, a hydrate thereof or a solvate thereof.

**3.** The use of a compound according to claim 1 or 2 which is (2R,3R,4S,5R)-2-hydroxymethyl-5-[6-[1-(5-chloropyridin-2-yl)-pyrrolidin-3(S)-ylamino]-purin-9-yl]-tetrahydrofuran-3,4-diol, (2R,3S,4R,5R)-2-hydroxymethyl-5-[6-[1-(5-trifluoromethylpyridin-2-yl)-pyrrolidin-3(R)-ylamino]-purin-9-yl]tetrahydrofuran-3,4-diol, (2R,3R,4S,5R)-2-hydroxymethyl-5-[6-[1-(5-trifluoromethylpyridin-2-yl)-pyrrolidin-3(S)-ylamino]-purin-9-yl]tetrahydrofuran-3,4-diol, (2R,3R,4S,5R)-2-hydroxymethyl-5-[6-[1-(4-trifluoromethylpyridin-2-yl)-pyrrolidin-3(S)-ylamino]-purin-9-yl]tetrahydrofuran-3,4-diol, (2R,3R,4S,5R) 2-hydroxymethyl-5-[6-[1-(5-bromopyridin-2-yl)-pyrrolidin-3(S)-ylamino]-purin-9-yl]-tetrahydrofuran-3,4-diol, (2R,3R,4S,5R)-2-hydroxymethyl-5-(6-(1-(4-nitrophenyl)-pyrrolidin-3(S)-ylamino) -purin-9-yl) tetrahydrofuran-3,4-diol, (2R,3R,4S,5R)-2-hydroxymethyl-5-[6-(5'-trifluoromethyl-3,4,5,6-tetrahydro-2H-[1,2']-bipyridinyl-3-yl)-purin-9-yl]tetrahydrofuran-3,4-diol, (2R,3R,4S,5R)-2-hydroxymethyl-5-[6-(phenylpyrrolidin-3(S)-ylamino)-purin-9-yl]tetrahydrofuran-3,4-diol, (2R,3R,4S,5R)-2-hydroxymethyl-5-[6-(1-pyridin-2-ylpyrrolidin-3(S)-ylamino]-purin-9-yl]tetrahydrofuran-3,4-diol, (2R,3R,4S,5R)-2-hydroxymethyl-5-[6-[1-(4-chlorophenyl)-pyrrolidin-3(S)-ylamino]-purin-9-yl]-tetrahydrofuran-3,4-diol, (2R,3R,4S,5R)-2-hydroxymethyl-5-[6-[1-(5-methylpyridin-2-yl)-pyrrolidin-3(S)-ylamino]-purin-9-yl]tetrahydrofuran-3,4-diol, (2R,3R,4S,5R)-2-hydroxymethyl-5-[6-[1-(5-thiophen-2-ylpyridin-2-yl)-pyrrolidin-3(S)-ylamino]-purin-9-yl]tetrahydrofuran-3,4-diol, (2R,3R,4S,5R)-2-hydroxymethyl-5-[6-[1-(5-methylmercaptopyridin-2-yl)-pyrrolidin-3(S)-ylamino]-purin-9-yl]tetrahydrofuran-3,4-diol, (2R,3R,4S,5R)-2-hydroxymethyl-5-[6-[1-(6-methoxypyrimidin-4-yl)pyrrolidin-3(S)-ylamino]-purin-9-yl]tetrahydrofuran-3,4-diol, (2R,3R,4S,5R)-2-hydroxymethyl-5-[6-[1-(6-chloropyrimidin-4-yl)pyrrolidin-3(S)-ylamino]-purin-9-yl]-tetrahydrofuran-3,4-diol, (2R,3R,4S,5R)-2-hydroxymethyl-5-[6-[1-(6-chloropyridazin-3-yl)pyrrolidin-3-ylamino]-purin-9-yl]-tetrahydrofuran-3,4-diol, (2R,3R,4S,5R)-2-methoxymethyl-5-[6-[1-(5-trifluoromethylpyridin-2-yl)-pyrrolidin-3(S)-ylamino]-purin-9-yl]tetrahydrofuran-3,4-diol, (1S,2R,3R,5R)-3-hydroxymethyl-5-[6-[1-(4-nitrophenyl)piperidin-4-yl]-purin-9-yl]cyclopentane-1,2-diol, (1S,2R,3R,5R)-3-hydroxymethyl-5-[6-((3S)-pyrrolidin-3-ylamino)-purin-9-yl] cyclopentane-1,2-diol dihydrochloride, (1S,2R,3R,5R)-3-hydroxymethyl-5-[6-[1-(4-nitrophenyl)pyrrolidin-3-ylamino]-purin-9-yl]cyclopentane-1,2-diol, (1S,2R,3R,5R)-3-hydroxymethyl-5-[6-[1-(5-trifluoromethylpyridin-2-yl)pyrrolidin-3(R)-ylamino]-purin-9-yl]cyclopentane-1,2-diol, (1S,2R,3R,5R)-3-hydroxymethyl-5-[6-((3R)-pyrrolidin-3-ylamino)-purin-9-yl] cyclopentane-1,2-diol, (1S,2R,3R,5R)-3-hydroxymethyl-5-[6-[1-(5-trifluoromethylpyridin-2-yl)pyrrolidin-3(S)-ylamino]-purin-9-yl]cyclopentane-1,2-diol, (1S,2R,3R,5R)-5-[6-[1-(5-bromopyridin-2-yl)pyrrolidin-3(S)-ylamino]-purin-9-yl]-3-hydroxymethylcyclopentane-1,2-diol, (1S,2R,3R,5R)-5-[6-[1-(5-chloropyridin-2-yl)pyrrolidin-3(S)-ylamino]-purin-9-yl]-3-hydroxymethylcyclopentane-1,2-diol, (1S,2R,3R,5R)-3-hydroxymethyl-5-[6-[1-(4-trifluoromethylpyridin-2-yl)pyrrolidin-3(S)-ylamino]-purin-9-yl]cyclopentane-1,2-diol, (1S,2R,3R,5R)-3-hydroxymethyl-5-[6-[1-(pyridin-2-yl)pyrrolidin-3(S)-ylamino]-purin-9-yl]cyclopentane-1,2-diol, (1S,2R,3R,5R)-3-hydroxymethyl-5-[6-[1-(quinolin-3-yl)pyrrolidin-3(S)-ylamino]-purin-9-yl]cyclopentane-1,2-diol, (1S,2R,3R,5R)-3-hydroxymethyl-5-[6-[1-S-(4-nitrophenyl)-pyrrolidin-3(S)-ylamino]-purin-9-yl]cyclopentane-1,2-diol, (1S,2R,3R,5R)-5-[6-[1-(4,5-bistrifluorpyridin-2-yl)pyrrolidin-3(S)-ylamino]-purin-9-yl]-3-hydroxymethylcyclopentane-1,2-diol, (1S,2R,3R,5R)-3-methoxymethyl-5-[6-[1-(5-trifluoromethylpyridin2-yl)pyrrolidin-3(S)-ylamino]-purin-9-yl]cyclopentane-1,2-diol, (1S,2R,3R,5R)-3-hydroxymethyl-5-[6-[1-(phenyl)-pyrrolidin-3(S)-ylamino]-purin-9-yl]cyclopentane-1,2-diol, 4-[3(S)-[9-(2,3-dihydroxy-4-hydroxymethylcyclopentyl)-9H-purin-6-ylamino]pyrrolidin-1-yl] benzonitrile, (1S,2R,3R,5R)-3-hydroxymethyl-5-[6-[1-(isoquinolin-1-yl)pyrrolidin-3(S)-ylamino]-purin-9-yl]cyclopentane-1,2-diol, (1S,2R,3R,5R)-5-[6-[1-(6-bromoquinolin-2-yl)pyrrolidin-3(S)-ylamino]-purin-9-yl]-3-hydroxymethylcyclopentane-1,2-diol, (1S,2R,3R,5R)-5-[6-[1-(4-chlorophenyl)pyrrolidin-3(S)-ylamino]-purin-9-yl]-3-hydroxymethylcyclopentane-1,2-diol, (1S,2R,3R,5R)-3-[6-[1-(3-chloro-5-trifluoromethylpyridin-2-yl)pyrrolidin-3(S)-ylamino]-purin-9-yl]-3-methoxymethylcyclopentane-1,2-diol, (1 S,2R,3R,5R)-5-[6-[1-(6-chloropyrimidin-4-yl) pyrrolidin-3(S)-ylamino]-purin-9-yl]-3-hydroxymethylcyclopentane-1,2-diol,(1S,2R,3R,5R)-5-[6-[1-(6-chloropyrimidin-4-yl)pyrrolidin-3(S)-ylamino]-purin-9-yl]-3-hydroxymethylcyclopentane-1,2-diol,(1S,2R,3R,5R)-5-[6-[1-(6-chloropyrimidin-4-yl)pyrrolidin-3(S)-ylamino]-purin-9-yl]-3-methoxymethylcyclopentane-1,2-diol, (1S,2R,3R,5R)-5-[6-[1-(6-chloropyridazin-3-yl)pyrrolidin-3(S)-ylamino]-purin-9-yl]-3-hydroxymethylcyclopentane-1,2-diol, (1S,2R,3R,5R)-3-methoxymethyl-5-[6-[1-(6-methoxypyrimidin-4-yl)pyrrolidin-3(S)-ylamino]-purin-9-yl]cyclopentane-1,2-diol, (1S,2R,3R,5R)-5-[6-[1-(6-chloropyridazin-3-yl)pyrrolidin-3(S)-ylamino]-purin-9-yl]-3-methoxymethylcyclopentane-1,2-diol, (1S,2R,3R,5R)-5-(-[6-[1-(4-trifluoromethylphenyl)-pyrrolidin-3(S)-ylamino]-purin-9-yl]-3-hydroxymethylcyclopentane-1,2-diol, (1S,2R,3R,5R)-5-[6-[1-(5-bromopyridin-2-yl)pyrrolidin-3(S)-ylamino]-purin-9-yl]-3-methoxymethylcyclopentane-1,2-diol, (1S,2R,3R,5R)-5-[6-[1-(5-chlorpyridin-2-yl)pyrrolidin-3(S)-ylamino]-purin-9-yl]-3-methoxymethylcyclopentane-1,2-diol,(1S,2R,3R,5R)-3-methoxymethyl-5-[6-[1-(4-trifluoromethylphenyl)pyrrolidin-3(S)-ylamino]-purin-9-yl]cyclopentane-1,2-diol, (1S,2R,3R,5R)-5-(-[6-[1-(4-chlorophenyl)-pyrrolidin-3(S)-ylamino]-purin-9-yl]-3-methoxymethylcyclopentane-1,2-diol, (1S,2R,3R,5R)-5-[6-[1-(3-chlorophenyl)-pyrrolidin-3(S)-ylamino] -purin-9-yl]-3-methoxymethylcyclopentane-1,2-diol, (1S,2R,3R,5R)-5-[6-[1-(3-chlorophenyl)-pyrrolidin-3(S)-ylamino] -purin-9-yl]-3-hydroxymethylcyclopentane-1,2-diol, (1S,2R,3R,5R)-3-methoxymethyl-5-[6-[1-phenylpyrrolidin-3-(S)-ylamino]-purin-9-yl]cyclopentan-1,2-diol, (1S,2R,3R,5R)-3-[6-(1-benzyl-pyrrolidin-3(S)-ylamino)purin-9-yl]5-hydroxymethylcyclopentane-1,2-diol, and (1S,2R,3R,5R)-3-[6-(1-benzyl-pyrrolidin-3(S)-ylamino)purin-9-yl]5-methoxymethylcyclopentane-1,2-diol; or a pharmaceutically acceptable salt thereof, a pharmaceutically acceptable prodrug thereof, an N-oxide thereof, a hydrate thereof or a solvate thereof.

**4.** The use of a compound according to claims 1 to 3 wherein Q is $CH_2$;

K is N:
T is

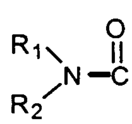

,

wherein $R_1$ is H and $R_2$ is lower alkyl;
A and B are hydroxy;
X is

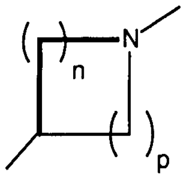

and n + p is 3 or 4;

or a pharmaceutically acceptable salt thereof, a pharmaceutically acceptable prodrug thereof, an N-oxide thereof, a hydrate thereof or a solvate thereof.

**5.** The use of a compound according to claim 4 which is (1S,2R,3S,4R)-2,3-dihydroxy-4-[6-[1-(5-trifluormethylpyridin-2-yl)pyrrolidin-3-ylamino]-purin-9-yl]cyclopentanecarboxylic acid ethylamide, (1S,2R,3S,4R)-2,3-dihydroxy-4-{6-[1-(5-trifluoromethyl-pyridin-2-yl)-pyrrolidin-3(S)-ylamino]-purin-9-yl}-cyclopentanecarboxylic acid-1(S)-methylpropylamide, and (1S,2R,3S,4R)-2,3-dihydroxy-4-{6-[1-(5-trifluoromethyl-pyridin-2-yl)-pyrrolidin-3(S)-ylamino]-purin-9-yl}-cyclopentanecarboxylic acid-1(R)-methylpropylamide; or a pharmaceutically acceptable salt thereof, a pharmaceutically acceptable prodrug thereof, an N-oxide thereof, a hydrate thereof or a solvate thereof.

**6.** The use of a compound according to claim 1 wherein Q is $CH_2$;

K is N;
T is hydroxymethyl or methoxymethyl;
A and B are hydroxy;
X is

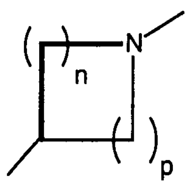

and n + p is 3 or 4;

or a pharmaceutically acceptable salt thereof, a pharmaceutically acceptable prodrug thereof, an N-oxide thereof, a hydrate thereof or a solvate thereof.

**7.** The use of a compound according to claim 6 which is (1S,2R,3R,5R)-3-hydroxymethyl-5-[6-[1-(4-nitrophenyl)piperidin-4-yl]-purin-9-yl]cyclopentane-1,2-diol, (1S,2R,3R,5R)-3-hydroxymethyl-5-[6-((3S)-pyrrolidin-3-ylamino)-purin-9-yl] cyclopentane-1,2-diol dihydrochloride, (1S,2R,3R,5R)-3-hydroxymethyl-5-[6-[1-(4-nitrophenyl)pyrrolidin-3-ylamino]-purin-9-yl]cyclopentane-1,2-diol, (1S,2R,3R,5R)-3-hydroxymethyl-5-[6-[1-(5-trifluoromethylpyridin-2-yl)pyrrolidin-3(R)-ylamino]-purin-9-yl]cyclopentane-1,2-diol, (1S,2R,3R,5R)-3-hydroxymethyl-5-[6-((3R)-pyrrolidin-3-ylamino)-purin-9-yl] cyclopentane-1,2-diol, (1S,2R,3R,5R)-3-hydroxymethyl-5-[6-[1-(5-trifluoromethylpyridin-2-yl)pyrrolidin-3(S)-ylamino]-purin-9-yl]cyclopentane-1,2-diol, (1S,2R,3R,5R)-5-[6-[1-(5-bromopyridin-2-yl)pyrrolidin-3(S)-ylamino]-purin-9-yl] -3-hydroxymethylcyclopentane-1,2-diol, (1S,2R,3R,5R)-5-[6-[1-(5-chloropyridin-2-yl)pyrrolidin-3(S)-ylamino]-purin-9-yl]-3-hydroxymethylcyclopentane-1,2-diol, (1S,2R,3R,5R)-3-hydroxymethyl-5-[6-[1-(4-trifluoromethylpyridin-2-yl)pyrrolidin-3(S)-ylamino]-purin-9-yl]cyclopentane-1,2-diol, (1S,2R,3R,5R)-3-hydroxymethyl-5-[6-[1-(pyridin-2-yl)pyrrolidin-3(S)-ylamino]-purin-9-yl]cyclopentane-1,2-diol, (1S,2R,3R,5R)-3-hydroxymethyl-5-[6-[1-(quinolin-3-yl)pyrrolidin-3(S)-ylamino]-purin-9-yl]cyclopentane-1,2-diol, (1S,2R,3R,5R)-3-hydroxymethyl-5-[6-[1-S-(4-nitrophenyl)-pyrrolidin-3(S)-ylamino]-purin-9-yl]cyclopentane-1,2-diol, (1S,2R,3R,5R)-5-[6-[1-(4,5-bistrifluorpyridin-2-yl)pyrrolidin-3(S)-ylamino]-purin-9-yl]-3-hydroxymethylcyclopentane-1,2-diol, (1S,2R,3R,5R)-3-methoxymethyl-5-[6-[1-(5-trifluoromethylpyridin2-yl)pyrrolidin-3(S)-ylamino]-purin-9-yl]cyclopentane-1,2-diol, (1S,2R,3R,5R)-3-hydroxymethyl-5-[6-[1-(phenyl)-pyrrolidin-3(S)-ylamino]-purin-9-yl]cyclopentane-1,2-diol, 4-[3(S)-[9-(2,3-dihydroxy-4-hydroxymethylcyclopentyl)-9H-purin-6-ylamino]pyrrolidin-1-yl] benzonitrile, (1S,2R,3R,5R)-3-hydroxymethyl-5-[6-[1-(isoquinolin-1-yl)pyrrolidin-3(S)-ylamino]-purin-9-yl]cyclopentane-1,2-diol, (1S,2R,3R,5R)-5-[6-[1-(6-bromoquinolin-2-yl)pyrrolidin-3(S)-ylamino]-purin-9-yl]-3-hydroxymethylcyclopentane-1,2-diol, (1S,2R,3R,5R)-5-[6-[1-(4-chlorophenyl)pyrrolidin-3(S)-ylamino]-purin-9-yl]-3-hydroxymethylcyclopentane-1,2-diol, (1S,2R,3R,5R)-3-[6-[1-(3-chloro-5-trifluoromethylpyridin-2-yl)pyrrolidin-3(S)-ylamino]-purin-9-yl]-3-methoxymethylcyclopentane-1,2-diol, (1S,2R,3R,5R)-5-[6-[1-(6-chloropyrimidin-4-yl)pyrrolidin-3(S)-ylamino]-purin-9-yl]-3-hydroxymethylcyclopentane-1,2-diol, (1S,2R,3R,5R)-5-[6-[1-(6-chloropyrimidin-4-yl)pyrrolidin-3(S)-ylamino]-purin-9-yl]-3-hydroxymethylcyclopentane-1,2-diol, (1S,2R,3R,5R)-5-[6-[1-(6-chloropyrimidin-4-yl)pyrrolidin-3(S)-ylamino]-purin-9-yl]-3-methoxymethylcyclopentane-1,2-diol, (1 S,2R,3R,5R)-5-[6-[1-(6-chloropyridazin-3-yl)pyrrolidin-3(S)-ylamino]-purin-9-yl]-3-hydroxymethylcyclopentane-1,2-diol, (1S, 2R,3R,5R)-3-methoxymethyl-5-[6-[1-(6-methoxypyrimidin-4-yl)pyrrolidin-3(S)-ylamino]-purin-9-yl]cyclopentane-1,2-diol, (1S,2R,3R,5R)-5-[6-[1-(6-chloropyridazin-3-yl)pyrrolidin-3(S)-ylamino]-purin-9-yl]-3-methoxymethylcyclopentane-1,2-diol, (1S,2R,3R,5R)-5-(-[6-[1-(4-trifluoromethylphenyl)-pyrrolidin-3(S)-ylamino]-purin-9-yl] -3-hydroxymethylcyclopentane-1,2-diol, (1S,2R,3R,5R)-5-[6-[1-(5-bromopyridin-2-yl)pyrrolidin-3(S)-ylamino]-purin-9-yl]-3-methoxymethylcyclopentane-1,2-diol, (1S,2R,3R,5R)-5-[6-[1-(5-chlorpyridin-2-yl)pyrrolidin-3(S)-ylamino]-purin-9-yl]-3-methoxymethylcyclopentane-1,2-diol, (1S,2R,3R,5R)-3-methoxymethyl-5-[6-[1-(4-trifluoromethylphenyl)pyrrolidin-3(S)-ylamino]-purin-9-yl]cyclopentane-1,2-diol, (1S,2R,3R,5R)-5-(-[6-[1-(4-chlorophenyl)-pyrrolidin-3(S)-ylamino]-purin-9-yl]-3-methoxymethylcyclopentane-1,2-diol, (1S,2R,3R,5R)-5-[6-[1-(3-chlorophenyl)-pyrrolidin-3(S)-ylamino] -purin-9-yl]-3-methoxymethylcyclopentane-1,2-diol, (1S,2R,3R,5R)-5-[6-[1-(3-chlorophenyl)-pyrrolidin-3(S)-ylamino] -purin-9-yl]-3-hydroxymethylcyclopentane-1,2-diol, (1S,2R,3R,5R)-3-methoxymethyl-5-[6-[1-phenylpyrrolidin-3-(S)-ylamino]-purin-9-yl]cyclopentan-1,2-diol, (1 S,2R,3R,5R)-3-[6-(1-benzyl-pyrrolidin-3(S)-ylamino)purin-9-yl]5-hydroxymethylcyclopentane-1,2-diol, and (1S,2R,3R,5R)-3-[6-(1-benzyl-pyrrolidin-3(S)-ylamino)purin-9-yl]5-methoxymethylcyclopentane-1,2-diol; or a pharmaceutically acceptable salt thereof, a pharmaceutically acceptable prodrug thereof, an N-oxide thereof, a hydrate thereof or a solvate thereof.

**8.** The use of a compound according to claim 6 which is (1S,2R,3R,5R)-3-hydroxymethyl-5-[6-[1-(5-trifluoromethylpyridin-2-yl)pyrrolidin-3(S)-ylamino]-purin-9-yl]cyclopentane-1,2-diol or (1S,2R,3R,5R)-3-hydroxymethyl-5-[6-[1-(4-trifluoromethylpyridin-2-yl)pyrrolidin-3(S)-ylamino]-purin-9-yl]cyclopentane-1,2-diol or a pharmaceutically acceptable salt thereof, a pharmaceutically acceptable prodrug thereof, an N-oxide thereof, a hydrate thereof or a solvate thereof.

**9.** The use of a compound according to claim 6 which is (1S,2R,3R,5R)-3-methoxymethyl-5-[6-[1-(5-trifluoromethylpyridin-2-yl)pyrrolidin-3(S)-ylamino]-purin-9-yl]cyclopentane-1,2-diol or (1S,2R,3R,5R)-3-methoxymethyl-5-[6-[1-(4-trifluoromethylpyridin-2-yl)pyrrolidin-3(S)-ylamino]-purin-9-yl]cyclopentane-1,2-diol or a pharmaceutically acceptable salt thereof, a pharmaceutically acceptable prodrug thereof, an N-oxide thereof, a hydrate thereof or a solvate thereof.

European Patent Office

**PARTIAL EUROPEAN SEARCH REPORT**

which under Rule 45 of the European Patent Convention shall be considered, for the purposes of subsequent proceedings, as the European search report

Application Number: EP 01 11 1651

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 99 24451 A (GLAXO GROUP LTD ;ELDRED COLIN DAVID (GB); PENNELL ANDREW MICHAEL K) 20 May 1999 (1999-05-20) | 1,2 | A61K31/52<br>A61P3/10 |
| Y | * claims 1,23 *<br>* page 31, line 19-21 *<br>* page 3, line 17-20 *<br>* page 7, line 24-26 *<br>* page 8, line 34 *<br>* See compound RN= 223756-98-7, page 35, line 19-20 * | 1-9 | |
| Y | WO 00 23447 A (MYERS MICHAEL R ;CHOI SLEDESKI YONG MI (US); PAULS HEINZ W (US); E) 27 April 2000 (2000-04-27)<br>* claims 1-9; examples *<br>* page 49, line 25-30 * | 1-9 | |
| Y | WO 98 01426 A (CHOI SLEDESKI YONG MI ;PAULS HENRY W (US); EWING WILLIAM R (US); M) 15 January 1998 (1998-01-15)<br>* page 60, line 30-36 *<br>* claims; examples * | 1-9 | |
| | -/-- | | |

TECHNICAL FIELDS SEARCHED (Int.Cl.7)

A61K

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| MUNICH | 23 November 2001 | Veronese, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

**INCOMPLETE SEARCH SHEET C**

Application Number
EP 01 11 1651

Claim(s) searched incompletely:
1-19

Reason for the limitation of the search:

The expression "prodrug" in claims 1-9 relates to an extremely large number of possible compounds/products/apparatus/methods. Support within the meaning of Article 84 EPC and/or disclosure within the meaning of Article 83 EPC is to be found, however, for only a very small proportion of the compounds/products/apparatus/methods claimed. In the present case, the claims so lack support, and the application so lacks disclosure, that a meaningful search over the whole of the claimed scope is impossible. Consequently, the search has been carried out for those parts of the claims which appear to be supported and disclosed, namely those parts relating to the compounds defined by the Markush formula in claim 1, with particular regard to the compounds specifically indicated in claims 3,5,7-9.

European Patent Office

# PARTIAL EUROPEAN SEARCH REPORT

Application Number
EP 01 11 1651

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | EP 0 490 818 A (SANDOZ LTD ;SANDOZ AG (DE); SANDOZ AG (AT)) 17 June 1992 (1992-06-17) * claim 5; examples * | 1-9 | |
| Y | EP 0 322 242 A (GLAXO GROUP LTD) 28 June 1989 (1989-06-28) * page 3, line 59; claims * | 1-9 | |
| A | EP 0 423 776 A (SEARLE & CO) 24 April 1991 (1991-04-24) * claim 7 * | 1 | |
| T | WO 01 40244 A (BELARDINELLI LUIZ ;CV THERAPEUTICS INC (US); PALLE VENKATA P (US);) 7 June 2001 (2001-06-07) * page 3, paragraphs * * See page 5, diabetic disorders * | 1 | TECHNICAL FIELDS SEARCHED (Int.Cl.7) |

EPO FORM 1503 03.82 (P04C10)

## ANNEX TO THE EUROPEAN SEARCH REPORT ON EUROPEAN PATENT APPLICATION NO. EP 01 11 1651

This annex lists the patent family members relating to the patent documents cited in the above-mentioned European search report. The members are as contained in the European Patent Office EDP file on
The European Patent Office is in no way liable for these particulars which are merely given for the purpose of information.

23-11-2001

| Patent document cited in search report | | Publication date | Patent family member(s) | | | Publication date |
|---|---|---|---|---|---|---|
| WO 9924451 | A | 20-05-1999 | AU | 1666599 | A | 31-05-1999 |
| | | | BR | 9813989 | A | 26-09-2000 |
| | | | CN | 1285844 | T | 28-02-2001 |
| | | | EE | 200000283 | A | 15-08-2001 |
| | | | WO | 9924451 | A2 | 20-05-1999 |
| | | | EP | 1030856 | A2 | 30-08-2000 |
| | | | HR | 20000277 | A1 | 31-10-2000 |
| | | | HU | 0004102 | A2 | 28-04-2001 |
| | | | NO | 20002359 | A | 05-07-2000 |
| | | | PL | 340873 | A1 | 12-03-2001 |
| | | | SK | 6702000 | A3 | 18-01-2001 |
| | | | TR | 200001937 | T2 | 21-11-2000 |
| WO 0023447 | A | 27-04-2000 | AU | 6410799 | A | 08-05-2000 |
| | | | WO | 0023447 | A1 | 27-04-2000 |
| WO 9801426 | A | 15-01-1998 | AP | 903 | A | 24-11-2000 |
| | | | AU | 3645497 | A | 02-02-1998 |
| | | | BG | 103135 | A | 30-11-1999 |
| | | | BR | 9710156 | A | 10-08-1999 |
| | | | CN | 1228770 | A | 15-09-1999 |
| | | | CZ | 9900024 | A3 | 12-05-1999 |
| | | | EP | 0912520 | A1 | 06-05-1999 |
| | | | HU | 9903815 | A2 | 28-04-2001 |
| | | | JP | 2000514801 | T | 07-11-2000 |
| | | | NO | 990063 | A | 08-03-1999 |
| | | | PL | 331036 | A1 | 21-06-1999 |
| | | | SK | 2299 | A3 | 09-10-2000 |
| | | | WO | 9801426 | A1 | 15-01-1998 |
| EP 0490818 | A | 17-06-1992 | DE | 4039060 | A1 | 11-06-1992 |
| | | | AT | 157007 | T | 15-09-1997 |
| | | | AU | 647216 | B2 | 17-03-1994 |
| | | | AU | 8888791 | A | 11-06-1992 |
| | | | CA | 2056967 | A1 | 08-06-1992 |
| | | | DE | 69127350 | D1 | 25-09-1997 |
| | | | EP | 0490818 | A1 | 17-06-1992 |
| | | | EP | 0774259 | A1 | 21-05-1997 |
| | | | FI | 915734 | A | 08-06-1992 |
| | | | HU | 61567 | A2 | 28-01-1993 |
| | | | IE | 914248 | A1 | 17-06-1992 |
| | | | IL | 100241 | A | 08-12-1995 |
| | | | JP | 9110700 | A | 28-04-1997 |
| | | | JP | 4290895 | A | 15-10-1992 |
| | | | NO | 178545 | B | 08-01-1996 |
| | | | NO | 954076 | A | 09-06-1992 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 01 11 1651

This annex lists the patent family members relating to the patent documents cited in the above-mentioned European search report. The members are as contained in the European Patent Office EDP file on
The European Patent Office is in no way liable for these particulars which are merely given for the purpose of information.

23-11-2001

| Patent document cited in search report | Publication date | Patent family member(s) | Publication date |
|---|---|---|---|
| EP 0490818 A | | NZ 240873 A | 27-09-1993 |
| | | PL 166094 B1 | 31-03-1995 |
| | | PT 99690 A ,B | 30-10-1992 |
| | | SG 44937 A1 | 19-12-1997 |
| | | ZA 9109658 A | 27-06-1993 |
| | | CZ 9200131 A3 | 17-02-1993 |
| | | MX 9200443 A1 | 01-01-1993 |
| | | NO 920564 A | 27-01-1993 |
| | | NO 964775 A | 27-01-1993 |
| | | SK 13192 A3 | 13-09-1995 |
| EP 0322242 A | 28-06-1989 | AT 315488 A | 15-02-1994 |
| | | AU 2740188 A | 29-06-1989 |
| | | AU 8589491 A | 12-12-1991 |
| | | BE 1002167 A5 | 28-08-1990 |
| | | CA 1320195 A1 | 13-07-1993 |
| | | CH 677495 A5 | 31-05-1991 |
| | | CN 1035295 A ,B | 06-09-1989 |
| | | CZ 9104039 A3 | 14-04-1993 |
| | | DE 3843609 A1 | 06-07-1989 |
| | | DE 3856154 D1 | 07-05-1998 |
| | | DE 3856154 T2 | 27-08-1998 |
| | | DK 713488 A | 24-06-1989 |
| | | EP 0322242 A2 | 28-06-1989 |
| | | ES 2012927 A6 | 16-04-1990 |
| | | FI 885943 A ,B, | 24-06-1989 |
| | | FR 2629715 A1 | 13-10-1989 |
| | | FR 2663936 A1 | 03-01-1992 |
| | | GB 2212498 A ,B | 26-07-1989 |
| | | GR 1000307 B | 12-05-1992 |
| | | HK 1009650 A1 | 04-06-1999 |
| | | HU 48903 A2 | 28-07-1989 |
| | | IE 61302 B | 19-10-1994 |
| | | IL 88765 A | 04-04-1993 |
| | | IT 1224840 B | 24-10-1990 |
| | | JP 1203400 A | 16-08-1989 |
| | | JP 2736088 B2 | 02-04-1998 |
| | | LU 87414 A1 | 07-07-1989 |
| | | MX 14331 A | 01-12-1993 |
| | | NL 8803140 A | 17-07-1989 |
| | | NO 885719 A ,B, | 26-06-1989 |
| | | NZ 227485 A | 25-06-1991 |
| | | PH 25932 A | 19-12-1991 |
| | | PL 276697 A1 | 19-02-1990 |
| | | PT 89328 A ,B | 29-12-1989 |
| | | SE 8804609 A | 21-12-1988 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 01 11 1651

This annex lists the patent family members relating to the patent documents cited in the above-mentioned European search report.
The members are as contained in the European Patent Office EDP file on
The European Patent Office is in no way liable for these particulars which are merely given for the purpose of information.

23-11-2001

| Patent document cited in search report | | Publication date | Patent family member(s) | | | Publication date |
|---|---|---|---|---|---|---|
| EP 0322242 | A | | SG | 44701 | A1 | 19-12-1997 |
| | | | SU | 1826971 | A3 | 07-07-1993 |
| | | | RU | 2060996 | C1 | 27-05-1996 |
| | | | US | 5032583 | A | 16-07-1991 |
| | | | YU | 234188 | A1 | 31-12-1990 |
| | | | ZA | 8809593 | A | 27-09-1989 |
| EP 0423776 | A | 24-04-1991 | US | 5055569 | A | 08-10-1991 |
| | | | CA | 2027999 | A1 | 20-04-1991 |
| | | | EP | 0423776 | A2 | 24-04-1991 |
| | | | IE | 903748 | A1 | 24-04-1991 |
| | | | JP | 3133995 | A | 07-06-1991 |
| | | | PT | 95629 | A | 13-09-1991 |
| WO 0140244 | A | 07-06-2001 | US | 6258793 | B1 | 10-07-2001 |
| | | | AU | 1937201 | A | 12-06-2001 |
| | | | AU | 2055201 | A | 12-06-2001 |
| | | | AU | 4138601 | A | 12-06-2001 |
| | | | WO | 0140244 | A1 | 07-06-2001 |
| | | | WO | 0140245 | A1 | 07-06-2001 |
| | | | WO | 0140799 | A2 | 07-06-2001 |
| | | | US | 2001008883 | A1 | 19-07-2001 |

EPO FORM P0459

For more details about this annex : see Official Journal of the European Patent Office, No. 12/82